# EUROPEAN PATENT SPECIFICATION

(11) **EP 1 503 802 B1**
(45) Date of publication and mention of the grant of the patent: **14.12.2005**
(21) Application number: 03728863.6
(22) Date of filing: 13.05.2003
(51) Int. Cl.: A61K 48/00, C08G 73/02

(54) **CONTROLLABLY DEGRADABLE POLYMERIC BIOMOLECULE OR DRUG CARRIER AND METHOD OF SYNTHESIZING SAID CARRIER**
KONTROLLIERTES ABBAUBARES POLYMER-BIOMOLEKÜL ODER WIRKSTOFFTRÄGER UND VERFAHREN ZUR SYNTHESE DIESES TRÄGERS
EXCIPIENT DE MEDICAMENTS OU DE BIOMOLECULES POLYMERE DONT LA DEGRADATION EST CONTROLABLE ET PROCEDE DE SYNTHESE DE CET EXCIPIENT

(30) Priority: 14.05.2002 US 378164 P; 11.10.2002 US 270788
(43) Date of publication of application: 09.02.2005
(73) Proprietor: NITTO DENKO CORPORATION, Ibaraki-shi, Osaka 567-8680 (JP)
(72) Inventor: YU, Lei, Carlsbad, CA 92008 (US); DU, Fusheng, Oceanside, CA 92054 (US); JI, Shouping, Oceanside, CA 92054 (US); MATSUMOTO, Kenji, San Diego, CA 92127 (US)
(74) Representative: Miles, John Stephen
(86) International application number: PCT/US2003/015003
(87) International publication number: WO 2003/097107

(56) References cited:
- WO-A-02/22174
- WO-A-97/45069
- US-A1- 2002 052 443
- US-B1- 6 319 516
- LYNN DAVID M ET AL: "ACCELERATED DISCOVERY OF SYNTHETIC TRANSFECTION VECTORS: PARALLEL SYNTHESIS AND SCREENING OF A DEGRADABLE POLYMER LIBRARY" JOURNAL OF THE AMERICAN CHEMICAL SOCIETY, AMERICAN CHEMICAL SOCIETY, WASHINGTON, DC, US, vol. 123, no. 33, 22 August 2001 (2001-08-22), pages 8155-8156, XP002197770 ISSN: 0002-7863 cited in the application

## Description

### FIELD OF THE INVENTION

The invention relates to a novel method for synthesizing a controllably degradable polymeric carrier molecule for biomedical application, such as biomolecule delivery, diagnostic imaging composition delivery, sensitizer composition delivery, and tissue engineering. More particularly, the invention relates to a controllably degradable polymer backbone and method of synthesizing polymers for use in delivery ofbiomolecules, such as nucleic acids, proteins, peptides, and drugs to cells, tissues, or to an individual in need of treatment.

### BACKGROUND OF THE INVENTION

The primary concern in gene therapy is gene delivery. Gene delivery systems are designed to protect and control the location of a gene within the body by affecting the distribution and access of a gene expression system to the target cell, and/or recognition by a cell-surface receptor, followed by intracellular trafficking and nuclear translocation *(Friedmann, T. The Development of Human Gene Therapy. Cold Spring Harbor Laboratory Press. San Diego, 1999).*

Interest in polymeric gene carriers is growing due to the limitations of viral vectors and cationic lipid-based gene carrier systems. Polymers are macromolecules that provide many exciting opportunities for the design of novel delivery systems of small molecular drugs, proteins, peptides, oligonucleotides and genes. In such systems, much greater flexibility can be achieved simply by varying the composition of the mixture, the polycation molecular mass, polycation architecture (linear, randomly branched, dendrimer, block and graft copolymer) and through modification of the polycation backbone by the introduction of side chains or other functional molecules, such as sugars, peptides, and antibodies (*Pouton CW, Seymour LW. Key issues in non-viral gene delivery. Adv. Drug Deliv. Rev. 2001 Mar 1;46(1-3):187-203).* Low immunogenicity or a lack thereof is another advantage over lipid-based gene carriers, which allows polymers to be a biocompatible material for application in patients.

The cationic polymers commonly used as gene carrier backbones are poly(L-lysine) (PLL), polyethyleneimine (PEI), chitosan, PAMAM dendrimers, and poly(2-dimethylamino)ethyl methacrylate (pDMAEMA).

Poly(L-lysine)-based polymers, pioneered in 1987, were used for gene delivery by employing a targeting ligand, e.g. asialoorosomucoid and folate to facilitate receptor-mediated uptake *(Wu, GY., and Wu, CH. Receptor-mediated in vitro gene transformation by a soluble DNA carrier system. J Biol Chem. 1987 Apr 5;262(10):4429-32; Wu, GY., and Wu, CH. Receptor-mediated gene delivery and expression in vivo. J Biol Chem. 1988 Oct 15;263(29):14621-4; Mislick KA, Baldeschwieler JD, Kayyem JF, Meade TJ. Transfection of folate-polylysine DNA complexes: evidence for lysosomal delivery. Bioconjug Chem. 1995 Sep-Oct;6(5):512-5).* It has been demonstrated that PLL/DNA complexes are internalized into cells as a result of the interaction of a ligand displayed at the surface of the complex with the receptor *(Wagner E, Zenke M, Cotten M, Beug H, Birnstiel ML. Transferrin-polycation conjugates as carriers for DNA uptake into cells. Proc Natl Acad Sci U S A. 1990 May; 87(9): 3410-4).* PLL-mediated gene transfer efficiency was also improved by employing lysosomatotropic agents (such as chloroquinine) or inactivated adenovirus, or peptide derived from Haemophilus Influenza envelope proteins to facilitate PLL/DNA complex release from the endosomes *(Wagner E, Plank C, Zatloukal K, Cotten M, Birnstiel ML. Influenza virus hemagglutinin HA-2 N-terminal fusogenic peptides augment gene transfer by transferrin-polylysine-DNA complexes: toward a synthetic virus-like gene-transfer vehicle. Proc Natl Acad Sci U S A. 1992 Sep 1;89(17):7934-8; Curiel DT, Wagner E, Cotten M, Birnstiel ML, Agarwal S, Li CM, Loechel S, Hu PC. High-efficiency gene transfer mediated by adenovirus coupled to DNA-polylysine complexes. Hum Gene Ther. 1992 Apr;3(2):147-54).* It is clear that without the use of either targeting ligands or endosome lytic reagents, gene transfer is poor with PLL polyplexes alone because PLL is composed only of primary amine. On the other hand, high molecular weight PLL showed significant toxicity to the cells.

Unlike PLL, both high molecular weight branched and linear polyethyleneimine (PEI) show efficient gene transfer efficiencies without the need for endosomolytic or targeting agents *(Boussif O, Lezoualc'h F, Zanta MA, Mergny MD, Scherman D, Demeneix B, Behr JP. A versatile vector for gene and oligonucleotide transfer into cells in culture and in vivo: polyethylenimine. Proc Natl Acad Sci U S A. 1995 Aug 1;92(16):7297-301).* Positively charged PEI polyplexes are endocytosed by cells, and PEI is also believed to facilitate endosomal escape due to its high density of secondary amines and tertiary amines. Unfortunately, higher molecular weight PEI has also been reported to be toxic to cells, which severely limits the potential for using PEI as a gene delivery tool in applications to human patients.

A range of polyamidoamine dendrimers has been studied as gene-delivery systems *(Eichman JD, Bielinska AU, Kukowska-Latallo JF, Baker JR Jr. The use of PAMAM dendrimers in the efficient transfer of genetic material into cells. Pharm. Sci. Technol. Today. 2000 Jul;3(7):232-245).* Terminal amino groups bind DNA by electrostatic means, forming positively charged complexes, which are taken up by endocytosis. There are advantages associated with the star shape of the polymer, as DNA appears to interact with the surface primary amines only, leaving the internal tertiary amines available to assist endosomal escape of the dendrimer-gene complex. Unfortunately, dendrimers have also been reported to be toxic to cells, which is the major limitation for its application in human patients. In addition, only polyamidoamine dendrimers with high generation showed practicable gene transfection efficiency, but the cost of preparing these polymers is very high.

The primary concern regarding gene carrier applications in medical gene therapy is safety and the potential of harm to cells, and then transfection efficiency. The large molecular weight cationic polymers described above that are required for efficient gene delivery usually show the inherited drawback of being toxic to the cells. On the other hand, although the low molecular weight cationic polymers or oligomers usually show less or no cytotoxicity, they also showed no significant gene transfection efficiencies. One of the strategies to solve this conflict is to synthesize a biodegradable cationic polymer that will be degraded to small molecules after the genes have been delivered into nucleic of the desired cells.

Recently, it has been reported that gene carriers made with degradable cationic polymers successfully transfer genes into mammalian cells with dramatically decreased cytotoxicity *(Lim YB, Kim SM, Lee Y, Lee WK, Yang TG, Lee MJ, Suh H, Park JS, J., Cationic Hyperbranched Poly(amino ester): A Novel Class of DNA Condensing Molecule with Cationic Surface, Biodegradable Three-Dimensional Structure, and Tertiary Amine Groups in the Interior, J. Am. Chem. Soc., 123 (10), 2460 -2461, 2001*). However, the lower gene transfer efficiency compared to non-degradable polymeric backbones may be due to the rapid degradation of these polymers in aqueous solution resulting in rapidly lost gene transfer efficiency during gene delivery reagent preparation or before the gene are delivered into the cells. The difficulty of controlling degradation rate and synthesizing biodegradable cationic polymers limits these polymeric gene carrier applications in *in vivo* gene delivery and in clinical patients.

To improve the transfection efficiency of low molecular weight PEI, Gosselin et al. *(Gosselin, Micheal A., Guo, Menjin, and Lee, Robert J. Efficient Gene Transfer Using Reversibly Cross-Linked Low Molecular Weight Polyethylenimine. Bioconjugate Chem. 2001. 12:232-245),* reported that the high molecular weight PEI could be achieved by using disulfide-containing linkers, Dithiobis(succinimidylpropionate) (DSP) and Dimethyl-3,3'-dithiobispropionimidate-2HCl (DTBP) and the resulting polymers showed comparable gene transfection efficiency and lower cytotoxicity. Since the cytoplasmic environment is markedly reducing, it is reasonable to expect that disulfide bonds introduced via cross-linking reagents will be reduced within the cytoplasm, resulting in the breakdown of PEI conjugates before genes are delivered into nucleus in which DNA transcription occurs. However, the disulfide-containing linkers used by Gosselin et al. are expensive, which makes large-scale preparation of this system difficult and undesirable. The polymers with disulfide-containing linkers are only degraded under reducing conditions, which limits polymer applications in other conditions. Furthermore, Gosselin et al. only discloses the use of branched PEI-800 Da, which may still show cytotoxicity if a large amount of the polymers are used in human body. In addition, by Gosselin's method, it is difficult to obtain polymers having significant gene transfer efficiency if the starting materials are low molecular weight cationic compounds (such as pentaethylenehexamine, N-(2-aminoethyl)-1,3-propanediamine).

Lynn, et al. *(Lynn, David A.; Anderson, Daniel G.; Putnam, David; and Langer, Robert. Accelerated Discovery of Synthetic Transfection Vectors: Parallel Synthesis and Screening of a Degradable Polymer Library. J. Am*. *Chem. Soc. 2001, 123, 8155-8156.)* describes a method of synthesizing biodegradable cationic polymers using diacrylates as linker molecules between cationic compounds. However, the resulting polymers are linear and have a low cationic density, which is insufficient to condense DNA. Synthesis of these polymers requires days to complete and the amount of effective product, which can be used in gene delivery, is low. More than one hundred cationic polymers were produced according to the methods of Lynn et al., but only two of these polymers showed effective gene transfection efficiency. These factors make the preparation of high molecular weight polymers by this method difficult to achieve.

### Summary of the Invention

There is a need for polymeric transfection vectors which are high molecular weight cationic polymers for efficiently delivering genetic materials, but which are controllably degradable in order to minimize cytotoxicity and cell damage. Although the majority of the description describes degradable polymers, this does not exclude the use of substantially non-degradable polymers.

One embodiment of the invention is a method of synthesizing controllably degradable cationic polymers, as well as a variety of biodegradable polymers. Biomolecules, such as nucleic acids and peptides, as well as synthetic drugs and other molecules can be conjugated to or complexed by the polymer, thus providing a delivery mechanism for the molecules of interest. Time- and spatial-controlled degradation of the polymers provides for highly efficient transfection of eukaryotic cells, particularly higher eukaryotic cells, with the molecules of interest while minimizing cell damage.

A further embodiment provides a simple method for transforming lower molecular weight cationic compounds or oligomers into efficient transfection materials with low cytotoxicity. In a preferred embodiment, one synthesis step completes the whole synthesis procedure under mild conditions and very short time. Therefore, it is easily scaled up for manufacturing and laboratory use at very low cost, since most of the starting materials for this synthesis method are commercially available.

Furthermore, the polymer synthesis method is highly effective. Transfection efficiency observed for polymers according to the preferred embodiment is high relative to other commercial polymeric gene carriers mediated transfection.

The polymer synthesis methods described herein are flexible in regard to the types of molecules which can be used to make high molecular weight polymers. Any cationic oligomer or compound with at least three amine groups could be used as a starting material to make useful polymeric gene carrier reagents with the addition of a linker molecule. The linker molecules in this invention contain hydrolyzable bonds. They may also contain other physically, biologically or chemically controllably cleavable bonds, such as reducible bonds, a peptide with enzyme specific cleavage sites, or physically or chemically sensitive bonds, such as optically sensitive, pH sensitive, or sonic sensitive molecules. The degradation of polymers of the present invention may be achieved by methods including, but not limited to, hydrolysis, enzymatic digestion, sonication, and physical degradation methods, such as photolysis.

The polymer synthesis methods described herein provide a useful method to easily make a polymer library for optimization of the reaction conditions for specific applications. These methods can also be used to synthesize polymer libraries for designing and screening for a polymer that has the researcher's desired characteristics, such as a specific degradation rate.

The polymer synthesis method also provides a useful method to easily incorporate a peptide, a sugar or a protein into a synthesized polymer by simply cross-linking the cationic compound with a linker or linkers that contain the functional group(s) of interest. The ligands also can be introduced into the synthesized polymers by conventional methods, such as a disulfide-containing group. Nucleic acids, peptides, drugs, other functional groups etc. can be conjugated/bound to the polymers by any method known to those of skill in the art.

Further preferred embodiments include biodegradable cationic polymers with controllable degradation rates, which exhibit high gene transfection efficiencies and low cytotoxicities compared to commercially available transfection reagents, such as lipofectamine (Invitrogen) and SuperFect (Qiagen). The degradation of polymers synthesized by methods described herein can be easily controlled by simply adjusting the ratio of molecules in the polymer composition or by changing various linker molecules.

In accordance with one preferred embodiment, there is provided a degradable cationic polymer comprising a plurality of cationic molecules and at least one degradable linker molecule connecting said cationic molecules in a branched arrangement. The cationic molecules are selected from the group consisting of:
(i) a cationic compound of formula 1: 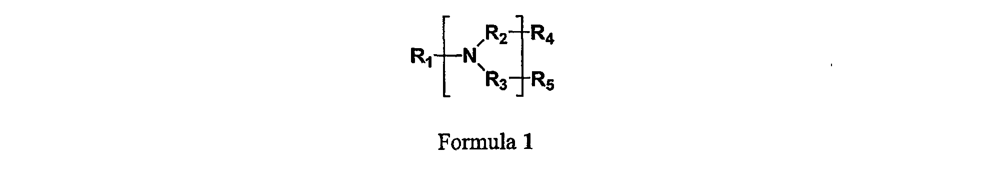 wherein:
   R₁ is a hydrogen atom, an alkyl or heteroalkyl group of 2 to 10 carbon atoms, an aryl or heteroaryl group with 5 to 30 atoms, or another Formula 1;
   R₂ is a hydrogen atom, an alkyl or heteroalkyl group of 2 to 10 carbon atoms, or aryl or heteroaryl group with 5 to 30 atoms;
   R₃ is a hydrogen atom, an alkyl or heteroalkyl group of 2 to 10 carbon atoms, or aryl or heteroaryl group with 5 to 30 atoms;
   R₄ is a hydrogen atom, an alkyl or heteroalkyl group of 2 to 10 carbon atoms, an aryl or heteroaryl group with 5 to 30 atoms, or another Formula 1;
   R₅ is a hydrogen atom, an alkyl or heteroalkyl group of 2 to 10 carbon atoms, an aryl or heteroaryl group with 5 to 30 atoms, or another Formula 1;
(ii) a cationic polyamino acid; and
(iii) a cationic polycarbohydrate;

The degradable linker molecule is represented by the formula:

CLₙ

wherein C is a spacer moiety that is a straight or branched alkyl or heteroalkyl group of 2 to 10 carbon atoms, or aryl or heteroaryl group with 5 to 30 atoms, may contain ether, ester, amide, imide, carbonyl groups with or without heteroatoms; L is an acrylate or methacrylate moiety, and n is an integer greater than or equal to two; and wherein C and L are bound covalently.

In accordance with another preferred embodiment, there is provided a biomaterial delivery system comprising at least one biomolecule, a degradable cationic polymer, as described above, having an affinity for the biomolecule, and at least one delivery enhancing agent, which can be internalized into a eukaryotic cell, that can facilitate receptor recognition, internalization, escape of the biomolecule from the endosome, nucleus localization, biomolecule release, or system stabilization in said eukaryotic cell.

In accordance with another preferred embodiment, there is provided a medical diagnostic system comprising an image contrast agent, the degradable cationic polymer, as described above, wherein the polymer has affinity for biomolecules, and a ligand, antibody, or agent of interest that recognizes a specific receptor of a eukaryotic cell, tissue, or organ, wherein said ligand, antibody, or agent is coupled with said degradable cationic polymer.

In accordance with another preferred embodiment, there is provided a pharmaceutical composition comprising a sensitizer agent that can be functionally triggered by light or other physical stimulators, and the degradable cationic polymer, as described above, wherein said polymer has affinity for biomolecules.

### Brief Description of the Drawings

As used in the following figures, different cationic polymers of the present invention are represented by CmLn, where Cm represents a cationic compound shown in Table 1 and Ln represents a linker molecule as shown in Table 2. For example, C1L4 represents a cationic polymer comprising cationic compound C1 of Table 1 and linker molecule L4 of Table 2.
Figure 1 is an illustration of the synthesis and degradation of the degradable cationic polymers. 'C' represents a cationic compound or a cationic oligomer, and 'L' represents a linker molecule.
Figure 2 illustrates the DNA binding affinity of oligomer C3, polymers C3L1 and C4L1. The polymer/DNA weight ratios are shown as 16, 8, 4, 2, 1 and 0.5, and free DNA (control without polymer) is shown as having the polymer/DNA weight ratio of 0. M indicates a 1 kb DNA molecular marker.
Figure 3 shows the electrophoresis pattern of some cationic oligomers and polymers. Lanes 1-3 are branch PEI, molecular weights 25KD, 10KD and 1.8KD, respectively. C3 is branched PEI_{600D}, PLL is poly-L-lysine with molecular weight of 30KD, and C3L1 is a polymer derived from C3 and L1.
Figure 4 illustrates the green fluorescent protein (GFP) gene transfection efficiency of different polymers at 24 hours after transfection, with Y axis indicates the polymer/DNA weight ratios used in transfection.
Figure 5 illustrates the GFP signal observed after 293 cells were transfected with GFP gene using the degradable cationic polymers and commercially available transfection reagents.
Figure 6 illustrates luciferase activity in 293 cells at 24 hours after pCMV-Luc gene transfection using various polymers.
Figure 7 illustrates cell survival after treatment with different polymer-DNA complexes.
Figure 8A illustrates the molecular weight change of C3L1 by means of agarose gel electrophoresis after C3L1 was incubated at 37°C for 0h(A), 6h(B), 12h(C), 1d(D), 2d(E), 3d(F), 4d(G), and 6d(H). Lane 1 contains branched PEI10K, and lane 2 contains branched PEI25K. Lane 3 and 4 contain C4 and C3, respectively.
Figure 8B illustrates the GFP gene transfection efficiency by using C3L1, after C3L1 was incubated at 37°C for 0h, 6h, 12h, 1d, 2d, 3d, 4d, and 6d.
Figure 9A illustrates the molecular weight change of C3L2 by means of agarose gel electrophoresis after C3L2 was incubated at 37°C for 0(A), 6h(B), 12h(C), 1d(D), 2d(E), 3d(F), 4d(G), and 6d(H). Lane 1 contains BPEI10K and lane 2 contains C3.
Figure 9B illustrates the GFP gene transfection efficiency by using C3L2 and C3L1 after their incubation at 37°C for 0h, 6h, 12h, 1d, 2d, 3d, 4d, and 6d.
Figure 10 illustrates the FITC signal after ODN was delivered by different polymers and illustrates the transfection efficiency of ODN by different polymers.
Figure 11 provides some examples of cationic molecules that may be used according to preferred embodiments described below. However, cationic molecules which may be used are not limited to these examples.
Figure 12 provides some examples of linker molecules that may be used according to preferred embodiments described below. However, linker molecules which may be used are not limited to these examples.
Figure 13 provides some examples of other amino-reactive residues that may be used besides (meth)acrylate groups, according to preferred embodiments described herein. However, reactive residues which may be used are not limited to these examples.
Figure 14 is a graphical representation of the percentage of cells into which the GFP gene has been transfected by different polymers (CmLn), and protein expression has occurred. Various combinations of cationic compounds and linkers were used to make the cationic carrier polymers. However, cationic carrier polymers which may be used are not limited to these examples.

### Detailed Description of the Preferred Embodiment

A degradable cationic polymer for delivery of biomolecules (nucleic acids, peptides, etc.), drugs, molecules used in medical imaging applications, sensitizing agents used in cancer treatments, and molecules used in tissue engineering is described herein. A method for synthesizing these polymers is also provided.

According to a preferred embodiment, a cationic oligomer or any molecules containing amino groups with more than three reactive sites can be used. These lower molecular weight cationic compounds or oligomers usually exhibit no or very low transfection efficiency when used as a carrier for gene or nucleic acid transport into cells. However, they do have low or no cytotoxicity in comparison to higher molecular weight carriers, which have high transfection efficiencies. Biodegradable cationic polymers typically exhibit low cytotoxicity, but also low transfection efficiency due to rapid degradation, making them less competitive against other carriers for gene transfer and other applications. These degradable cationic polymers improve transfection efficiency by linking low molecular weight cationic compounds or oligomers together with degradable linkers. The linker molecules may contain biologically, physically or chemically cleavable bonds, such as hydrolyzable bonds, reducible bonds, a peptide sequence with enzyme specific cleavage sites, pH sensitive, or sonic sensitive bonds. The degradation of these polymers may be achieved by methods including, but not limited to hydrolysis, enzyme digestion, and physical degradation methods, such as optical cleavage_(photolysis).

Additionally, the degradable polymers may be conjugated to or associated with a viral or non-viral protein to enhance transfection efficiency. For example, vesicular stomatitis virus G protein (VSVG) and other peptides or proteins which are known to those of skill in the art may be added to the polymers in order to improve transfection efficiency.

One of the most attractive features of the polymers described herein is that degradation of the polymers is controllable in terms of rate and site of polymer degradation, based on the type and structures of the linkers.

Cationic oligomers, such as low molecular weight polyethyleneimine (PEI), low molecular weight poly(L-lysine) (PLL), low molecular weight chitosan, and low molecular weight PAMAM dendrimers, can be used to make the polymers described herein. Furthermore, any molecule containing amines with more than three reactive sites can be used. Cationic compounds may be selected from, but are not limited to:
(i) a cationic compound of formula 1: 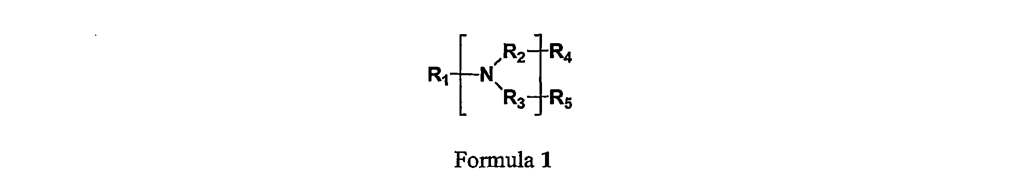 wherein:
   R₁ is a hydrogen atom, an alkyl or heteroalkyl group of 2 to 10 carbon atoms, an aryl or heteroaryl group with 5 to 3 0 atoms, or another Formula 1;
   R₂ is a hydrogen atom, an alkyl or heteroalkyl group of 2 to 10 carbon atoms, or aryl or heteroaryl group with 5 to 30 atoms;
   R₃ is a hydrogen atom, an alkyl or heteroalkyl group of 2 to 10 carbon atoms, or aryl or heteroaryl group with 5 to 30 atoms;
   R₄ is a hydrogen atom, an alkyl or heteroalkyl group of 2 to 10 carbon atoms, an aryl or heteroaryl group with 5 to 30 atoms, or another Formula 1;
   R₅ is a hydrogen atom, an alkyl or heteroalkyl group of 2 to 10 carbon atoms, an aryl or heteroaryl group with 5 to 30 atoms, or another Formula 1;
(ii) a cationic polyamino acid; and
(iii) a cationic polycarbohydrate;

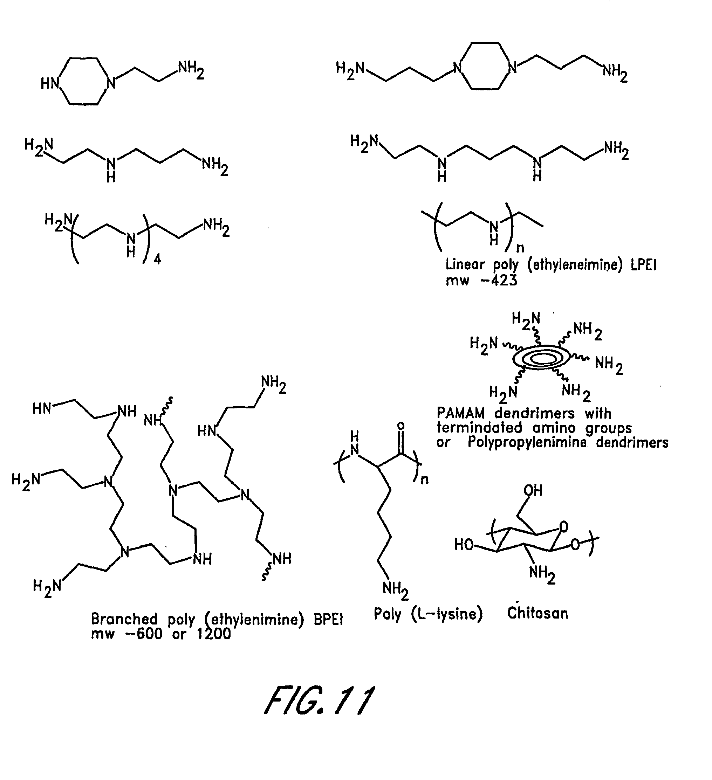

Examples of such cationic molecules include, but are not limited to, the cationic molecules shown in Figure 11 and Table 1.

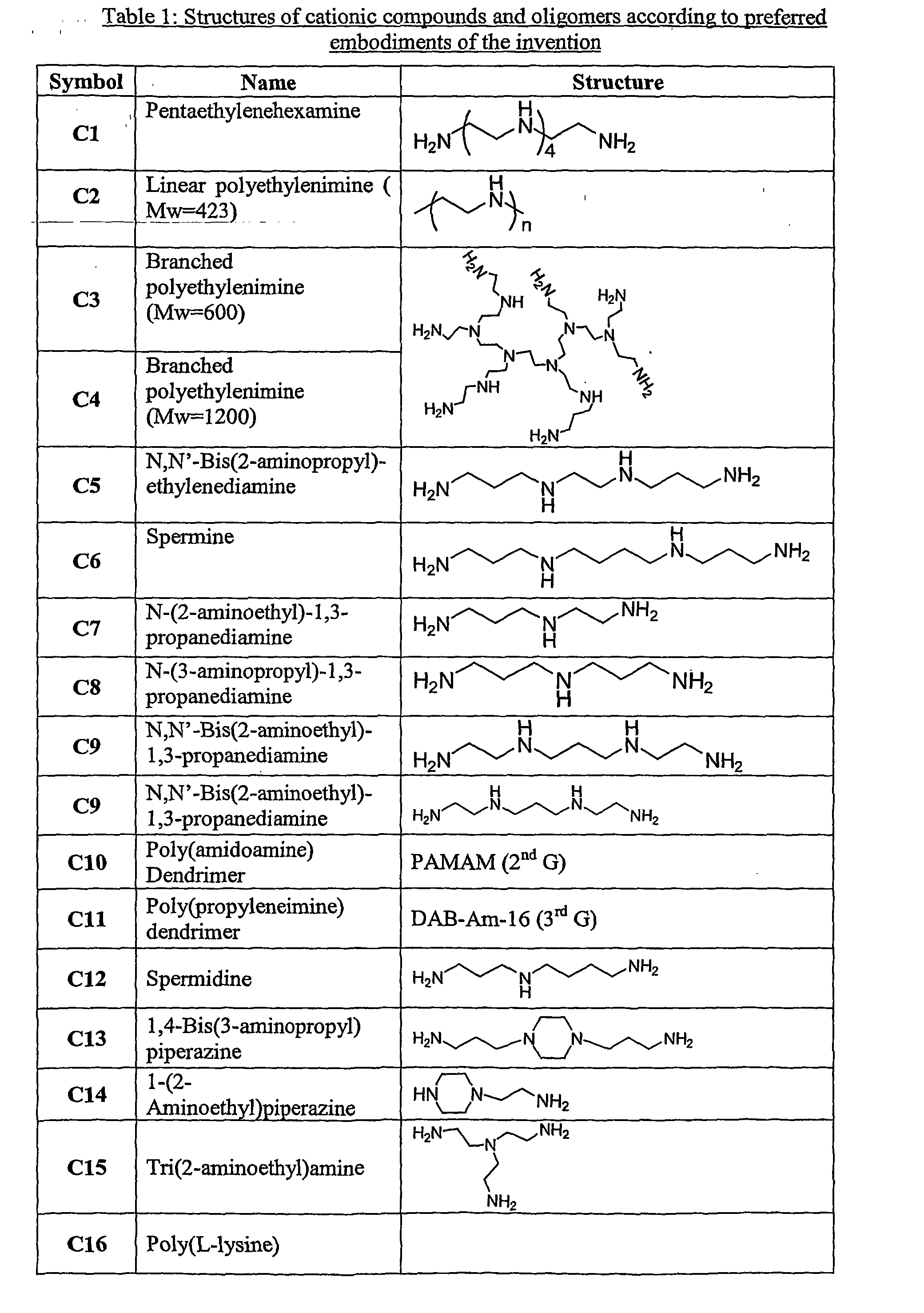

Cationic polymers used herein may comprise primary or secondary amino groups, which can be conjugated with active ligands, such as sugars, peptides, proteins, and other molecules. In a preferred embodiment, lactobionic acid is conjugated to the cationic polymers. The galactosyl unit provides a useful targeting molecule towards hepatocyte cells due to the presence of galactose receptors on the surface of the cells. In a further embodiment, lactose is conjugated to the degradable cationic polymers in order to introduce galactosyl units onto the polymer.

Degradable linking molecules include, but are not limited to, di- and multi-acrylates, di- and multi-methacrylates, di- and multi-acrylamides, di- and multi-isothiocyanates, di- and multi-isocyanates, di- and multi-epoxides, di- and multi-aldehydes, di- and multi-acyl chlorides, di- and multi-sulfonyl chlorides, di- and multi-halides, di- and multi-anhydrides, di- and multi-malemides, di- and multi-carboxylic acids, di- and multi-á-haloacetyl groups, and di- and multi-N-hydroxysuccinimide esters, which contain at least one biodegradable spacer. The following formula describes a linker which may be used according to preferred embodiments:

CLₙ

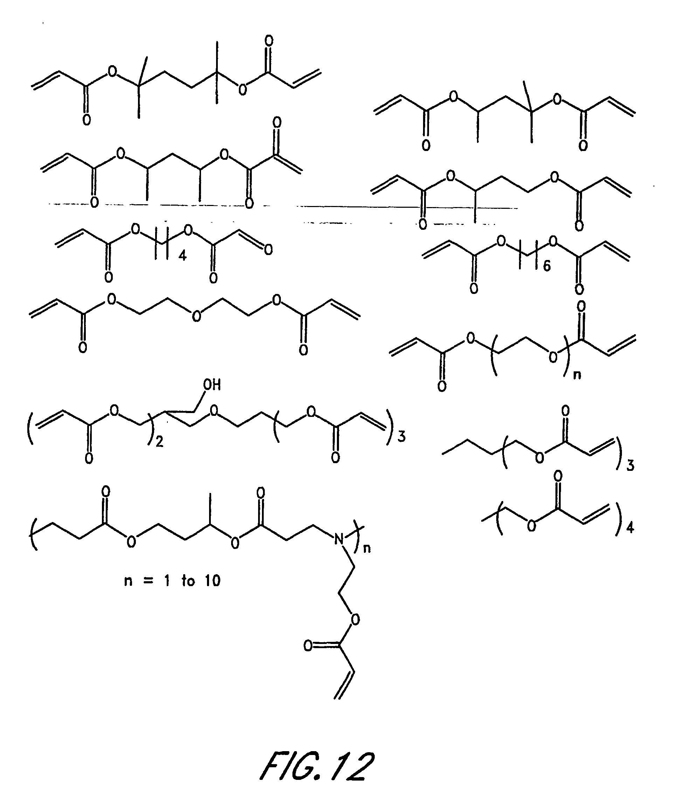

wherein C is a spacer moiety that is a straight or branched alkyl or heteroalkyl group of 2 to 10 carbon atoms, or aryl or heteroaryl group with 5 to 30 atoms, may contain ether, ester, amide, imide, carbonyl groups with or without heteroatoms; L is an acrylate or methacrylate moiety, and n is an integer greater than or equal to two; and wherein C and L are bound covalently. Several examples of linker molecules have been provided in Figure 12, however other embodiments of these molecules have been envisioned and have been described herein.

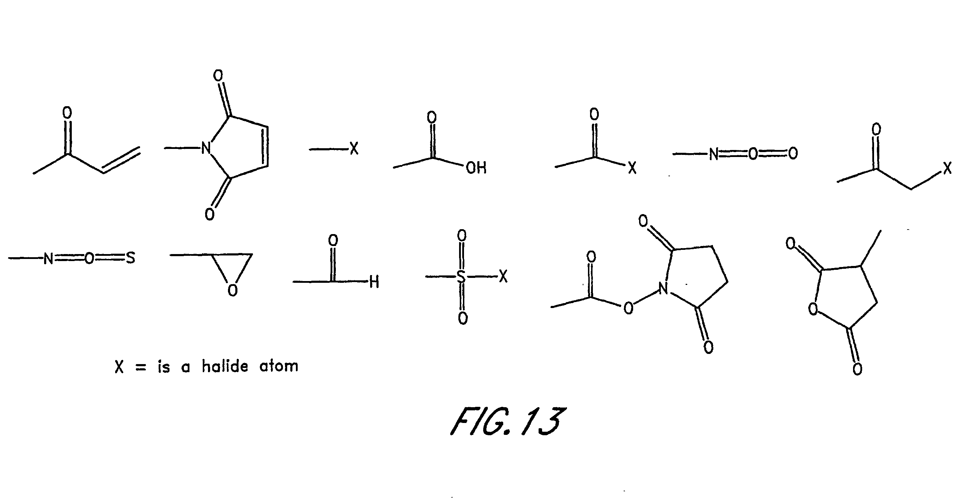

Several embodiments of reactive residues of the linker molecules have been illustrated in Figure 13, however these examples are not limiting to the scope of the invention. Reactive residues may be selected from, but are not limited to, acryloyl, maleimide, halide, carboxyl acylhalide, isocyanate, isothiocyanate, epoxide, aldehyde, sulfonyl chloride, and N-hydroxysuccinimide ester groups or combinations thereof. Other embodiments of the linker and cationic molecules have been disclosed herein. Table 2 contains linkers used in preferred embodiments of the invention.

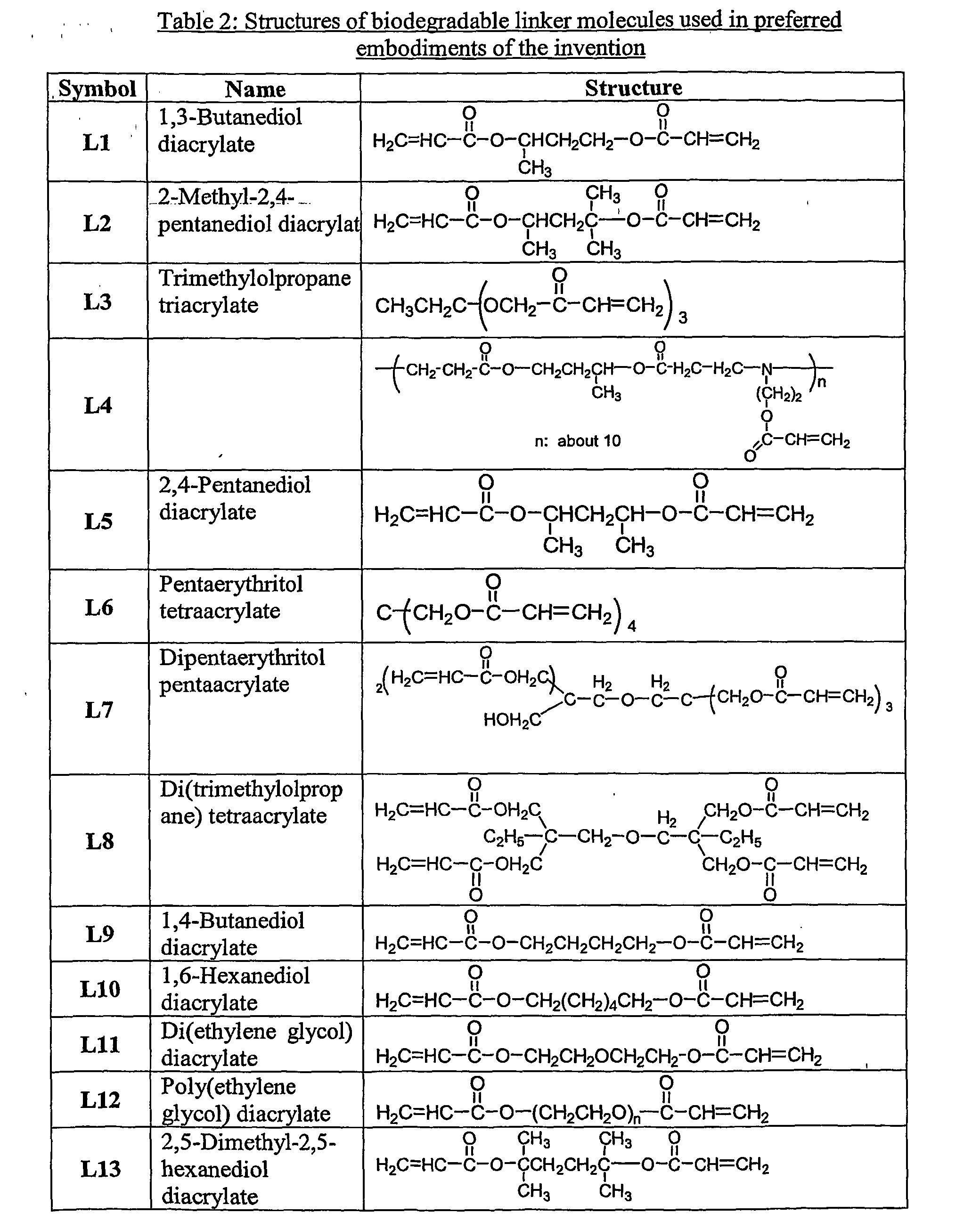

The degradation rates of the polymers can be controlled by changing the polymer composition, feed ratio, and the molecular weight of the polymers. For example, when linkers with bulkier alkyl groups are used as linkers, the polymers will degrade slower. Also, increasing molecular weight will cause a decrease in the degradation rate in some cases. Degradation rates of the polymers may be controlled by adjusting the ratio of cationic polymer to linker or by changing the various degradable linker molecules.

In a further embodiment of the present invention, non-degradable cationic polymers may be produced. The linker molecule(s) between the cationic compounds of these polymers is/are not degradable by the methods described herein.

Acrylate linkers are much cheaper than disulfide-containing linkers, because synthesis of the disulfide-containing linkers is more difficult. Acrylate linkers can be hydrolyzable in any water solution, therefore a polymer containing acrylate linkers can be degraded in various environments as long as it contains water. Thus, polymers containing acrylate linkers have broad applications compared to disulfide-linker-containing polymers. In addition, the degradation rate of polymers with disulfide-linkers are usually the same, but the degradation rate of polymers synthesized with acrylate linkers can vary depending on the different acrylate linkers used.

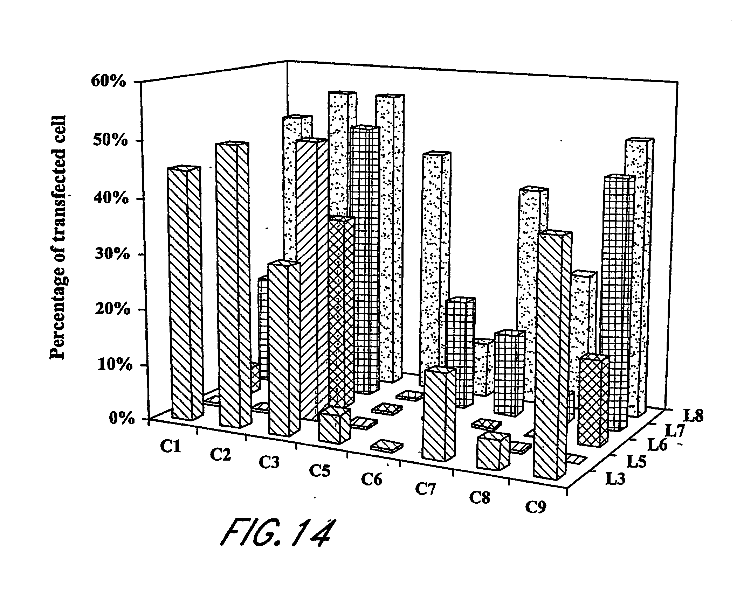

The synthesis methods described herein to make cationic polymers are simple and relatively low in cost. A library of biodegradable cationic polymers can be obtained using different combinations of cationic compounds or oligomers and linker molecules, or by changing ratios of cationic compounds to linkers. The physical and chemical properties of the polymers in this library can be adjusted by using different combinations of cationic compounds and linkers or changing the ratio of cationic compounds to linker molecules. The polymers of this library can be used as degradable gene carriers to introduce plasmid DNA and antisense oligo-DNA of interest into cells. The GFP transfection results as shown in Figure 14 indicate that more than 50% of these polymers can effectively deliver the GFP gene into cells and result in expression of the protein.

### Example 1

### Synthesis Overview

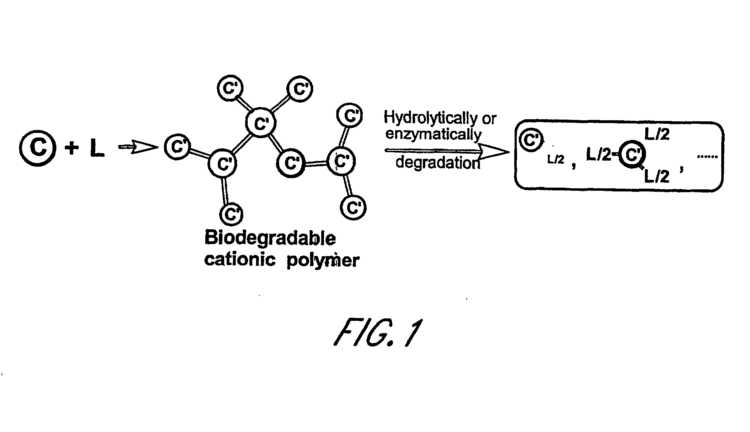

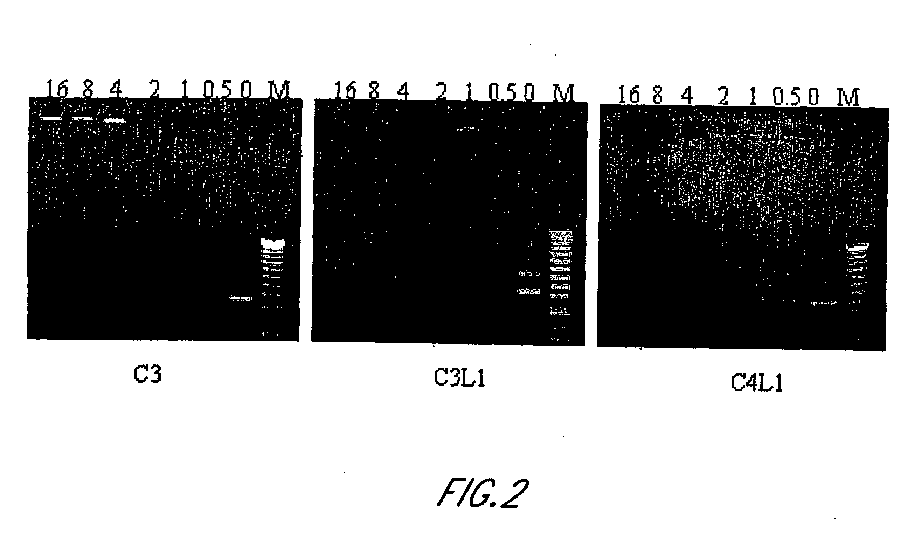

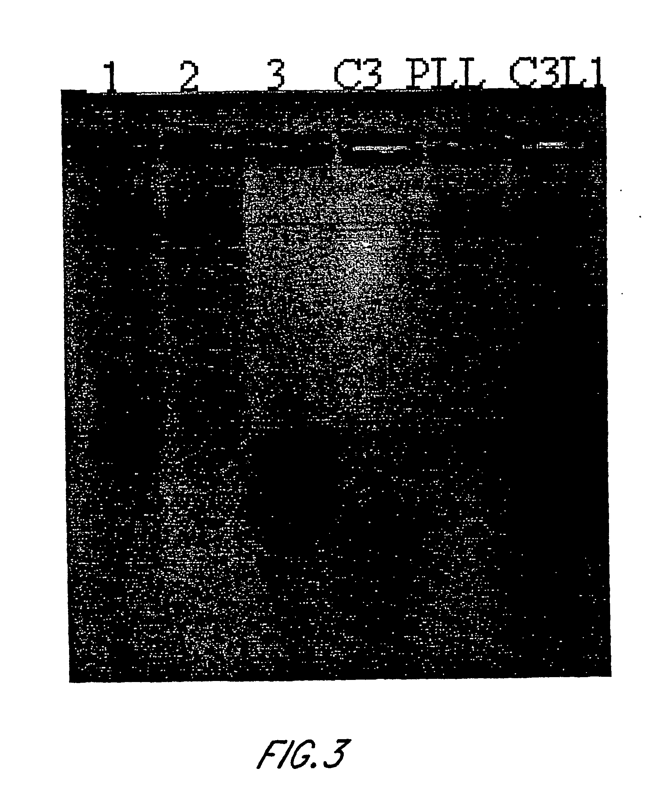

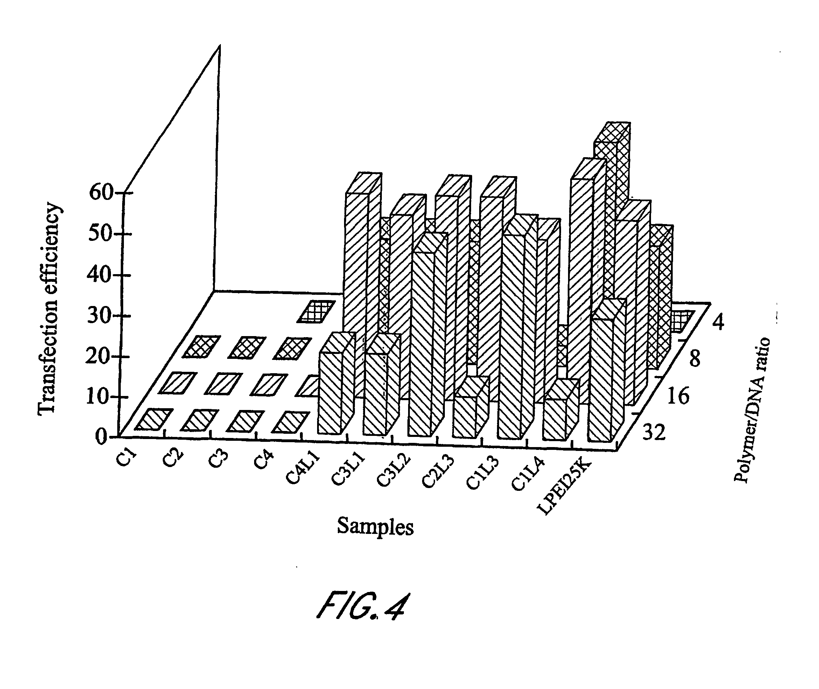

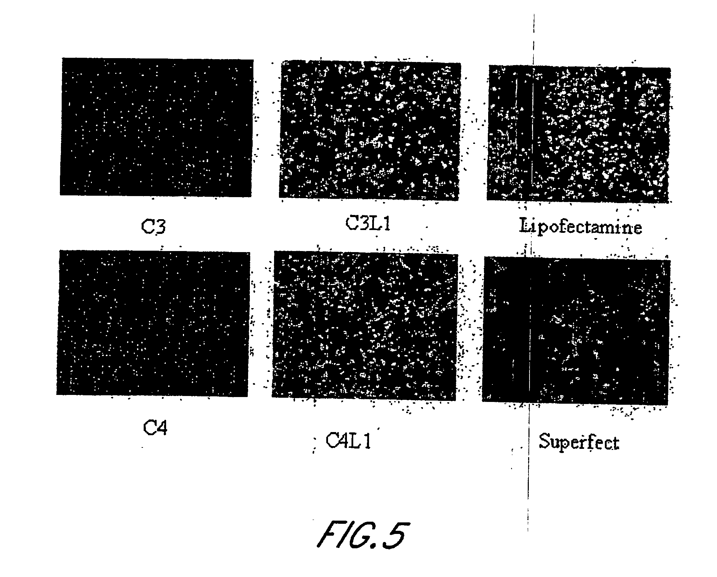

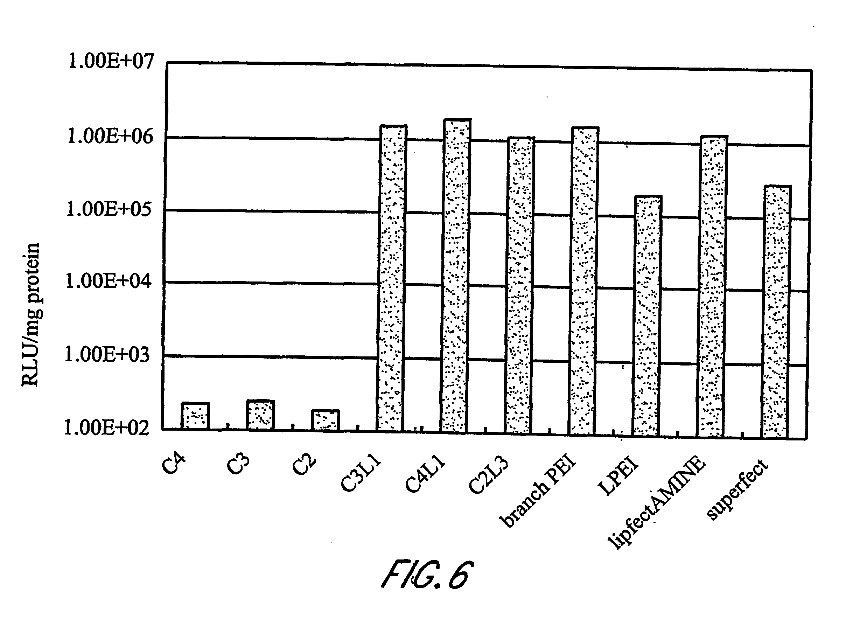

Synthesis of branched or slightly cross-linked biodegradable cationic polymers is illustrated in Figure 1. This synthesis method can be used for preparation of large libraries of branched or slightly crosslinked biodegradable cationic polymers. Degradation of the cationic polymers of the present invention is also illustrated.

In Figure 1, C represents an amine-containing cationic compound or oligomer with at least three reactive sites (for Michael addition reaction), and L represents a compound having at least two acrylate groups. The reaction between C and L takes place under very mild conditions in organic solvents After reaction, the polymers can be recovered by two different methods. In the first method, the polymers were recovered by direct removal of the solvents at reduced pressure. In the second method, the polymers were neutralized by adding acid, such as hydrochloric acid, and the neutralized polymers were recovered by filtration or centrifugation. Branched or slightly cross-linked, water soluble polymers with high molecular weight can be obtained by controlling the ratio of C to L, reaction time, reaction temperature, solvents, and concentration of the solutes.

### Example 2

### Polymers prepared by crosslinking cationic oligomers with diacrylate linkers, recovered by direct removing solvents

Synthesis of high molecular weight cationic polymers according to the present invention may be performed by a variety of methods know to those of ordinary skill in the art. The synthesis of a polymer which is derived from polyethylenimine oligomer with molecular weight of 600 (PEI-600) and 1,3-butanediol diacrylate (1,3-BDODA) is provided as a general procedure to serve as a model for other synthetic procedures involving similar compounds which can be used to synthesize a series of degradable cationic polymers. 0.44g of PEI-600 (Aldrich) was weighed and placed in a small vial, and 6 ml of methylene chloride was added. After the PEI-600 completely dissolved, 0.1g of 1,3-BDODA in 2 ml of methylene chloride was added slowly into the PEI solution while stirring. The reaction mixture was stirred for 10 hours at room temperature. After removing the organic solvent under reduced pressure, 0.55g of transparent, viscous liquid was obtained. ¹H-NMR spectrum indicated that the acrylic carbon-carbon double bond disappeared completely. The molecular weight of the obtained polymer was estimated by agarose gel electrophoresis. Other branched or slightly crosslinked, degradable cationic polymers derived from other cationic oligomers and other linkers having structures similar to those used herein were prepared in a similar manner.

### Example 3

### Polymers prepared by crosslinking cationic oligomers with diacrylate linkers, recovered after neutralization with acid

The synthesis of a polymer which is derived from PEI-600 and 1,6-hexanediol diacrylate (1,6-HDODA) is provided as a general procedure to serve as a model for other synthetic procedures involving similar compounds which can be used to synthesize a series of degradable cationic polymers. To a 20ml small vial, 0.43g of PEI-600 in 2ml of methylene chloride was added by using pipette or syringe. 0.23g (1.0mmol) of 1,6-HDODA were quickly added to the above PEI-600 solution under stirring. The concentration of PEI-600 in the reaction solution was adjusted to 0.1g/ml by adding more methylene chloride. The reaction mixture was stirred for 5 hours at room temperature (25°C). Then, the reaction mixture was neutralized by adding 2.5 ml of 4M HCl. The white precipitate was filtered, washed with methylene chloride, and dried at room temperature under reduced pressure. The obtained polymer was characterized with NMR spectrometer and agarose gel electrophoresis. Other cationic oligomers, such as polypropyleneimine and other polyalkyleneimines having structures similar to those used herein were used to prepare the other different degradable cationic polymers by in a similar manner.

### Example 4

### Polymers prepared by crosslinking cationic oligomers with multi-acrylate linkers

Acrylate type linkers with three or more than three acrylate groups can be used to crosslink the cationic oligomers as mentioned in examples 2 and 3. But, compared to diacrylate linkers, lower molar ratio of linker to cationic oligomer is needed when linkers with three or more acrylate groups are used. The crosslinking reaction of PEI-600 with trimethylolpropane triacrylate (TMOPTA) is provided as a general procedure to serve as a model for other synthetic procedures involving similar compounds. To a solution containing 0.43g of PEI-600 in 2ml of methylene chloride, 0.13g (0.44mmol) of TMOPTA in 2ml of methylene chloride was quickly added under stirring. The concentration of PEI-600 in the reaction solution was adjusted to 0.1g/ml by adding more CH₂Cl₂. The reaction mixture was stirred for 5 hours at room temperature (25°C), and the polymer was recovered by the same method as in example 3. Polymers prepared by crosslinking other polyalkyleneimines with other multi-acrylate linkers having structures similar to those used herein were prepared in a similar manner.

### Example 5

### Polymers prepared by crosslinking PAMAM oligomers with acrylate linkers

Poly(amido-amine) dendrimers (PAMAM) with terminated primary or secondary amino groups were used as cationic oligomers to prepare the degradable cationic polymers by the method of present invention. Mixed solvent of methanol and methylene chloride was used as a solvent in order to make a homogeneous solution. 0.1g of PAMAM (second generation, 0.39mmol of primary amino groups) was dissolved in a mixed solvent of 0.5ml of methanol and 1.0ml of methylene chloride. To this solution, 40mg of 1,3-BDODA in 1ml of methylene chloride were added under stirring. After stirring at 5°C for 10h, 0.25ml of 2M HCl were added to the reaction mixture. The polymer was recovered by centrifugation and dried under reduced pressure at room temperature. The polymers derived from PAMAM oligomer and other acrylate linkers having structures similar to those used herein were prepared in a similar manner.

### Example 6

### Polymers prepared by crosslinking poly(amino acid) oligomers with acrylate linkers

0.11g of poly(L-lysine) hydrobromide oligomer (Mw:1000-3000) and 50mg of triethylamine were dissolved in 1.0ml of dry DMSO. To the above solution, 42mg of 2,4-pentanediol diacrylate (2,4-PDODA) in 1.0ml of dry DMSO were added quickly. After stirring at room temperature for 6 hours, the pH value of the reaction mixture was adjusted to 4.5 by adding 0.5M of HCl aqueous solution. The polymer was purified through dialysis in HCl aqueous solution (pH: 4.0, 4°C) by using a tubing with MWCO of 3000, and recovered by freeze-drying method. The polymers derived from other poly(amino acid) oligomers, which contain more than three primary or secondary amino groups, and other acrylate linkers having structures similar to those used herein were prepared in a similar manner.

### Example 7

### Polymers prepared by crosslinking multi-amines with acrylate linkers

Besides the cationic oligomers as used from example 2 to example 6, multi-amines with low molecular weights can also be used to prepare the degradable cationic polymers by the crosslinking method of present invention. The crosslinking reaction of pentaethylenehexamine (PEHA) with di(trimethylolpropane)tetraacrylate (DTMOPTA) is provided as a general procedure to serve as a model for other synthetic procedures involving similar compounds. 0.23g of PEHA was weighed into a small vial containing 2ml of methylene chloride. After PEHA completely dissolved, 0.28g of DTMOPTA in 1 ml of methylene chloride was added slowly into the above solution under stirring. Another 2ml of methylene chloride were added. After stirring for 8 hours at room temperature, the reaction mixture was neutralized by adding 2 ml of 4M HCl. The polymers was recovered by direct removal of the organic solvents, and then dried at reduced pressure at room temperature. The polymers derived from other multi-amines and acrylate linkers having structures similar to those used herein were prepared in a similar manner.

### Example 8

### A library of degradable cationic polymers prepared by crosslinking cationic oligomers or compounds with acrylate linkers

Based on the procedures as described from example 2 to example 6, a library of branched or slightly cross-linked, water soluble, degradable cationic polymers were prepared from different cationic oligomers or compounds and different linkers. The cationic oligomers or compounds and the linkers used in the present invention are shown in, but are not limited to, Table 1 and Table 2, respectively, and the polymers prepared in the present invention are shown in, but are not limited to, Table 3. The properties of the polymers were adjusted by controlling the ratio of cationic compound to linker, reaction time, reaction temperature, solvents, and concentrations of the solutes. Some of these polymers were evaluated by GFP reporter gene transfection efficiency (Figure 11)

**Table 3**

| Polymers prepared by using different cationic compounds and linkers | | | | | | | | | | | | | |
|---|---|---|---|---|---|---|---|---|---|---|---|---|---|
| | **L1** | **L2** | **L3** | **L4** | **L5** | **L6** | **L7** | | **L9** | **L10** | **L11** | **L12** | **L13** |
| **C1** | *C1L1* | | *C1L3* | *C1L4* | *C1L5* | *C1L6* | *C1L7* | *C1L8* | | | | | |
| **C2** | *C2L1* | | *C2L3* | | *C2L5* | *C2L6* | *C2L7* | *C2L8* | | | | | |
| **C3** | *C3L1* | *C3L2* | | | | *C3L6* | *C3L7* | *C3L8* | *C3L9* | *C3L10* | *C3L11* | *C3L12* | *C3L13* |
| **C4** | *C4L1* | *C4L2* | | | | | | | *C4L9* | | *C4L11* | | |
| **C5** | *C5L1* | | *C5L3* | | *C5L5* | *C5L6* | *C5L7* | *C5L8* | | | | | |
| **C6** | | | *C6L3* | | | *C6L6* | *C6L7* | *C6L8* | | | | | |
| **C7** | | | *C7L3* | | *C7L5* | *C7L6* | *C7L7* | *C7L8* | | | | | |
| **C8** | *C8L1* | | *C8L3* | | *C8L5* | *C8L6* | *C8L7* | *C8L8* | | | | | |
| **C9** | *C9L1* | | *C9L3* | | *C9L5* | *C9L6* | *C9L7* | *C9L8* | | | | | |
| **C10** | *C10L1* | *C10L2* | | | | | | | | | | | |
| **C11** | *C11L1* | | *C11L3* | | | | | | | | | | |
| **C12** | | | *C12L3* | | | *C12L6* | *C12L7* | *C12L8* | | | | | |
| **C13** | | | *C13L3* | | | *C13L6* | *C13L7* | *C13L8* | | | | | |
| **C14** | *C14L1* | | *C14L3* | | *C14L5* | *C14L6* | *C14L7* | *C14L8* | | | | | |
| **C15** | *C15L1* | | *C15L3* | *C15L4* | | | | | | | | | |
| **C16** | | | | | *C16L5* | | | *C16L8* | | | | | |

### Example 9

### Substantially Non-degradable cationic polymers prepared by crosslinking cationic oligomers with diepoxide linkers

The synthesis of a polymer prepared by crosslinking PEI-600 with glycerol diglycidyl ether is provided as a general procedure to serve as a model for other synthetic procedures which can be used to prepare a series of non-degradable cationic polymers. 0.43g of PEI-600 and 0.37g of glycerol diglycidyl ether were dissolved in 7.0ml of methanol. The reaction solution was stirred at 40°C for 48h. After cooling to room temperature, 2.5 ml of 4M HCl (in dioxane) were added to the reaction solution, causing appearance of white precipitate. The polymers were recovered by centrifugation and dried at room temperature under reduced pressure. The obtained polymer was characterized with NMR spectrometer and agarose gel electrophoresis. Polymers prepared by crosslinking other cationic oligomers with other diepoxide linkers were prepared in a similar manner.

### Example 10

### Substantially Non-degradable cationic polymers prepared by crosslinking multi-amines with multi-epoxide linkers

The synthesis of a polymer prepared by crosslinking N,N'-Bis(2-aminopropyl)-ethylenediamine (BAPEDA) with trimethylolpropane triglycidyl ether (TMOPTE) is provided as a general procedure to serve as a model for other synthetic procedures involving similar compounds. 0.18g of BAPEDA and 0.24g of TMOPTE was dissolved in 3.0ml of methanol. The reaction solution was stirred at 35°C for 74h hours. After cooling to room temperature, 1.0 ml of 4M HCl (in dioxane) were added to the reaction solution, and the precipitated polymers were recovered by removing the solvents under reduced pressure. The obtained polymer was characterized with NMR spectrometer and agarose gel electrophoresis. Polymers prepared by crosslinking other multi-amines with other multi-epoxide linkers were prepared in a similar manner.

### Example 11

### Conjugation of galactosyl unit onto the cationic polymers

In one embodiment of the synthesis method, 102 mg of polymer C3L5 and 50 mg of lactobionic acid were added to 10 ml of water, which was adjusted to pH 5.5 by adding aqueous Na₂CO₃. Twenty-five (25) mg of 1-[3-dimethylamino)propyl]-3-ethyl carbodiimide was added under vigorous stirring. After stirring for five (5) hours at room temperature, the reaction solution was dialyzed in water (pH=3.5) for 24 hours. The galactose-conjugated polymer was recovered by freeze-drying and was characterized by NMR spectrometer.

### Example 12

### DNA retardation experiment

Binding and condensing DNA is the first step of the cationic polymer-mediated gene transfection process. A DNA retardation experiment is commonly used to determine polymer DNA binding affinity. The polymers with poor DNA binding capacity usually show low or no transfection efficiency. The experiment protocol is described as follows. Briefly, different ratios of polymer in 10µl DMEM (without serum and antibiotic) were added to 0.2µg green fluorescent protein (GFP) plasmid in 10µl DMEM (without serum and antibiotic) drop by drop and while being vortexed. The resulting complexes were placed at room temperature for 15 minutes prior to electrophoresis. Five microliters (5µl) of loading dye was added to each complex, and 15µl of each mixture was loaded into each well, respectively. DNA complexes were analyzed by electrophoresis in a 0.3% agarose gel with 0.04 M Tris-acetate buffer, pH 7.4, containing 1mM EDTA, at 100V for 30 minutes. DNA was visualized by UV illumination. In the electric field, free DNA ran out from the well, and plasmid bands could be observed in the gel (line 0 of each samples, in Fig. 2). If the plasmid was completely packed by polymer, its migration will be completely inhibited, and no bands in the gel would be observed on the gel. However if the plasmid is not bound by the polymer the plasmid will travel out of the well and the bound plasmid or a smear can be observed in the gel. In this experiment, the DNA binding affinity of the starting cationic compounds or oligomers, pentaethylenehexamine(C1), linear PEI 423Da(C2), branch PEI 600Da(C3) and branch PEI 1200Da(C4) was very weak, even when polymer/DNA ratio was 16:1. Plasmid still leaked (Fig. 2, C3). After cross-linking, all the polymers derived from the starting oligomers or cationic compounds according to the present invention showed high binding affinity. For example, DNA migration was completely inhibited by C3L1, when polymer/DNA ratio was 2:1 (Fig. 2, C3L1), and DNA migration was completely inhibited by C4L1 when polymer/DNA ratio was 4:1 (Fig. 2,-C4L1). The results in Figure 2 indicate that the polymers produced by the current invention increase DNA binding affinities.

### Example 13

### Estimate molecular weight of polymers by agarose gel electrophoresis

Molecular weight is a key issue in determining DNA binding affinity and transfection efficiency. Higher molecular weight is required for high DNA binding affinity, as well as transfection efficiency. One of the important advantages of present invention is that it can make low molecular weight cationic oligomers or cationic compounds into larger molecular weight polymers. To evaluate the molecular range of polymers synthesized by present invention, an agarose gel electrophoresis assay was conducted. In this experiment, polymers were dissolved in 150mM NaCl solution to 5mg/ml of final concentration. Twenty microliter (20µl) samples were taken and mixed with 2µl 50% glycerol and 1µl of Oregon red fluorescent dye for each well and loaded in 0.6% agarose gel in TAE buffer. Electrophoresis was performed at 100 volts for 30 min., and the polymer molecular weights were analyzed by visualization of the fluorescent dye under UV light or by visualization after commassie blue staining. The polymer migration rates were dependent on the size of polymers. Generally, the low molecular weight polymers migrate faster in the agarose gel than high molecular weight polymers. In these experiments, branched PEI_{25K}, PEI_{10K}, PEI_{1.8K} were used as polymer molecular weight standards in lane 1 to lane 3, respectively. Lane C3 is a cationic oligomer, PEI_{0.6K,} as a starting material for synthesis of polymer C3L1. The results showed that the molecular weight of polymer C3L1 is much higher than its starting material, C3, compared to the molecular weight standard (Fig. 3).

### Example 14

### In vitro transfection

Transfection protocols varied throughout these studies as indicated for individual experiments. The permanent cells (293 and HT1080 cells, ATCC) were plated in 24-well tissue culture plates (2X10⁵ cells/well for 293 cells and 8X10⁴ cells/well for HT1080) and incubated overnight in DMEM (Gibco) with 10% FBS (Gibco). The precise mixing order of the plasmid-polymer complex is an important parameter in the determining the outcome of transfection. For each well, an aliquot of 30ì1 DMEM containing different amounts of the polymers was added into 30-ì1 DMEM solution containing 0.61 g of plasmid DNA, e.g. pCMV-GFP plasmid DNA or pCMV-luc, drop by drop while vortexing. The polymer-DNA solutions were incubated for 15 min. at room temperature to allow the formation of DNA-polymer complexes. One hundred-fifty microliters (150ì1) DMEM medium containing 10%FBS and antibiotics were add to the DNA-polymer complex, and then the mixtures were added to the cells in individual wells after the cells were washed with PBS. Cells were incubated (37°C, 7.5% CO₂) for 3 hrs., and then the medium was changed to DMEM medium containing 10%FBS and 100U/ml Penicillin and 100µg/ml streptomycin. Twenty-four hours after transfection, GFP signal and firefly luciferase activities were detected using the methods described below. Lipofectamine and Superfect were used as positive control according to the protocol provided by manufacturer

### Novel Synthetic Polymer Mediated GFP Reporter Gene Transfection

Green fluorescent protein (GFP) gene was used in the first screening. After transfection, the GFP signal in cells were observed under a fluorescent microscope (Olympus, filter 515-550 nm). Cells were photographed using a 10X objective. The percentage of cells with GFP signal in transfected cultures was determined from counts of three fields for optimal cationic polymer amounts. It was found that the starting polymer, C1, C2, C3 and C4 showed almost no transfection efficiency. After cross-linking, the polymers derived from them showed high transfection efficiency. For example, the transfection efficiency of C4L1, C3L1, C3L2, C2L3, C1L3, C1LA were around 45% to 55%, better than LPEI 25KDa (about 45%).

Figure 5 shows the typical results derived from use of C3, C4 and C3L1, C4L1. C3 and C4 showed almost no GFP signal in the tested cells at 24h after transfection. C4L1 and C3L1 showed very bright GFP signal, which were comparable to commercial transfection reagent lipofectamine (Gibco), and better than another commercial transfection reagent, Superfect (Qiagen).

### Novel Synthetic Polymer Mediated Luciferase Reporter Gene Transfection

Measurement of luciferase activity was performed using a chemiluminescent assay following the manufacturer's instructions (Luciferase Assay System; Promega, Madison, WI, USA). Briefly, thirty hours after gene transfer, the cells were rinsed twice with PBS and then were lysed with lysis buffer (1% Triton X-100, 100 mM K₃PO₄, 2 mM dithiothreitol, 10% glycerol, and 2 mM EDTA pH 7.8) for 15 min at room temperature. A 10-ì 1 aliquot of cell lysate was then mixed with 50-ì1 of luciferase assay reagent with injector at room temperature in the luminometer. Light emission was measured in triplicate over 10 seconds and expressed as RLUs (relative light units). Relative light units (RLU) were normalized to the protein content of each sample, determined by Coommassie Protein Assay (Pierce, Rockford, Illinois). All the experiments were conducted in triplicate. The results of transfection of 293, HT1080 with pCMV-luc using various transfection reagents are presented in Fig. 6. The results showed that C4, C3 and C2 have no transfection efficiencies; the luciferase activities were similar to background. After cross-linking, C3L1, C4L1 and C2L3 derived from those low molecular weight cationic compounds showed high transfection efficiencies. The luciferase activity of C4L1 was 1.88×10⁶, higher than branched PEI 25K and lipofectamine (luciferase activity was 1.58 and 1.28×10⁶ respectively), and it had 7.9 fold higher luciferase activity compared to linear PEI 25K. The results indicate that by using the present invention method, the transfection efficiency could be significantly improved, resulting in an excellent method of making new transfection reagents.

### Example 15

### Novel Synthetic Polymer Toxicity to Cells

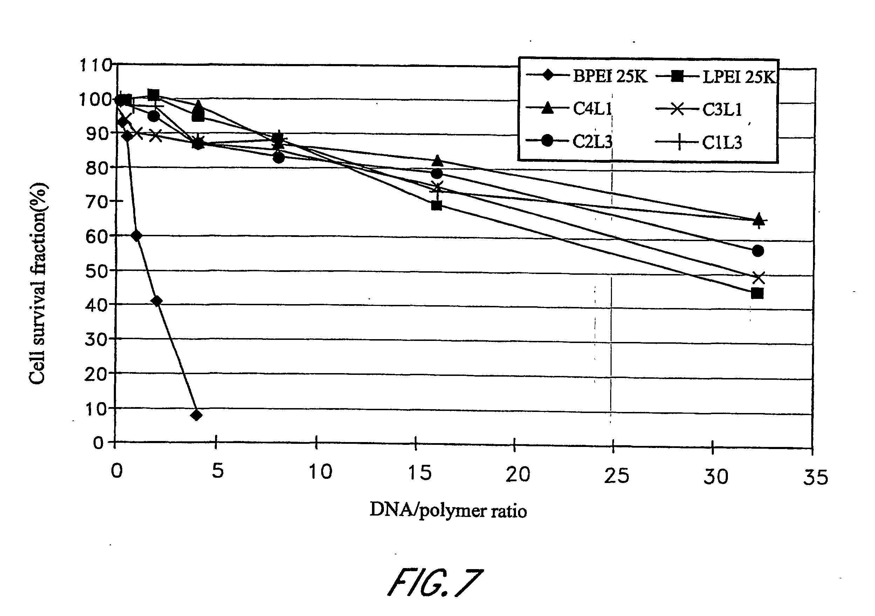

The cytotoxicities of cationic gene carriers on mammalian cells were evaluated using a 3-[4,5 dimethylthiazol-2-yl]-2,5-diphenyltetrazolium bromide (MTT) method. Briefly, HT1080 cells, 2 x 10⁴ cells/well or 4 x 10⁴ 293 cells, were seeded in 96-well plates and incubated for 16-24 hr. An aliquot of 15 µl DMEM, containing the polymers, was added drop by drop into 15 µl DMEM containing 0.3 µg plasmid and incubated at room temperature for 15 min to form polymeric-DNA complexes. Seventy-five microliters (75µl) of DMEM was added to the polymer-DNA complexes, and 50µl of the mixture was added to the cells and incubated (37° C, 7.5% CO₂) for 3h. The media was then removed and DMEM medium containing 10%FBS and 100U/ml Penicillin and 100µg/ml streptomycin were added. Following further incubation for 24 hrs, the media was removed and 10ì1 of MTT solution (5.0 mg/ml, Sigma) was added to each well, and incubated for 3 hrs. The medium was then removed and 200 i 1DMSO was added to dissolve the formazan crystals. The absorbance of the solution was measured at 570 nm. Cell viabilities was calculated using the equation: Viability (%) = {Abs_{570 (sample)} /Abss_{570 (control)}} x 100. All the experiments were conducted in triplicate. The results indicated that the cytotoxicity of C4L1, C3L1, C2L3, C1L3 were lower than branched PEI _{25K}, as many more cells survived following transfection; the cytotoxicity for these polymers was similar to or lower than cytotoxicity caused by LPEI 25K (Fig. 7). The results indicate that the present invention method can be used to easily obtain synthetic cationic polymers with high gene transfection efficiency and lower cytotoxicity.

### Example 16

### Controllable Degradation of Synthetic Polymers

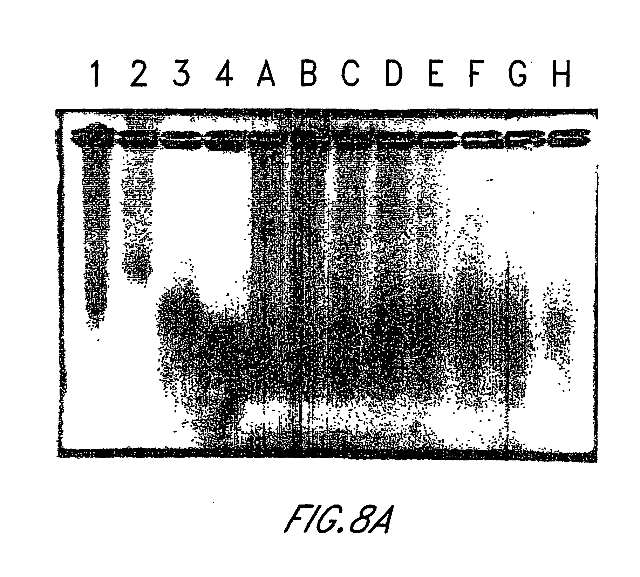

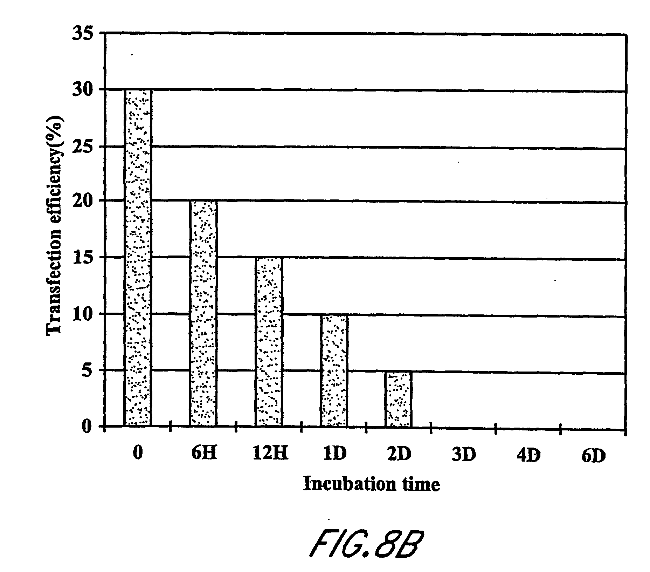

In order to evaluate the degradation of novel synthetic cationic polymer, C1L3 derived from PEI 600D, was incubated with PBS at 37°C for 6 hours (h), 12h, 1 day (d), 2d, 3d, 4,d and 6d. Polymer degradation was evaluated by the gel profile of the molecular weight analysis (Fig. 8A), as well as transfection efficiency (Fig. 8B) before and after incubation. The data from the molecular weight assay showed that before incubation the molecular weight of C1L3 was high compared to the standard polymer, PEI25K. After 6 hours of incubation at 37°C, polymers were degraded to low molecular weight oligomers, as indicated by the evidence that the polymer bands on the upper part of the gel gradually disappeared and a staining band at the bottom of the gel accumulated. Three days after incubation, most polymers appeared in low molecular areas, indicating that the polymers were almost completely degraded (Fig. 8A). The results were correlative with the results of transfection efficiency, which showed that 3 days after incubation at 37°C, transfection decreased from 30% to 0 (Fig. 8B).

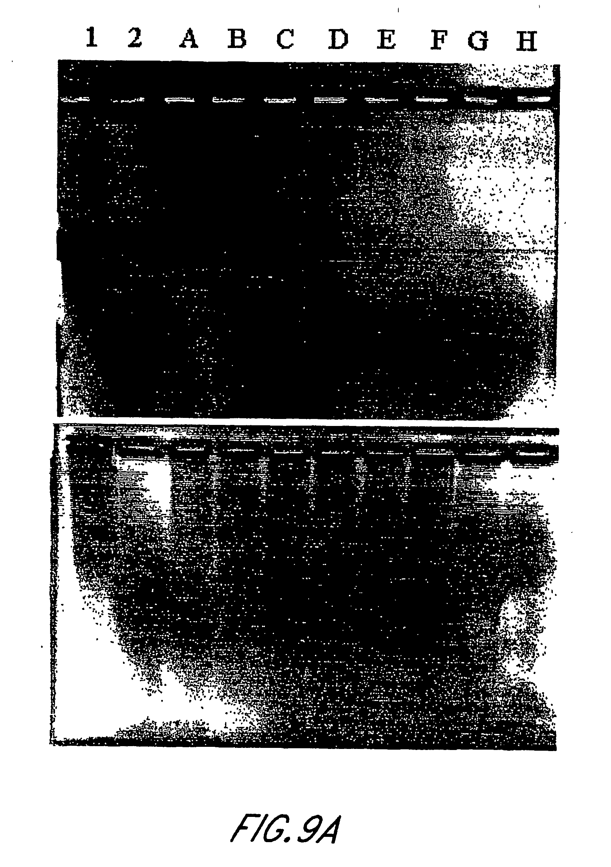

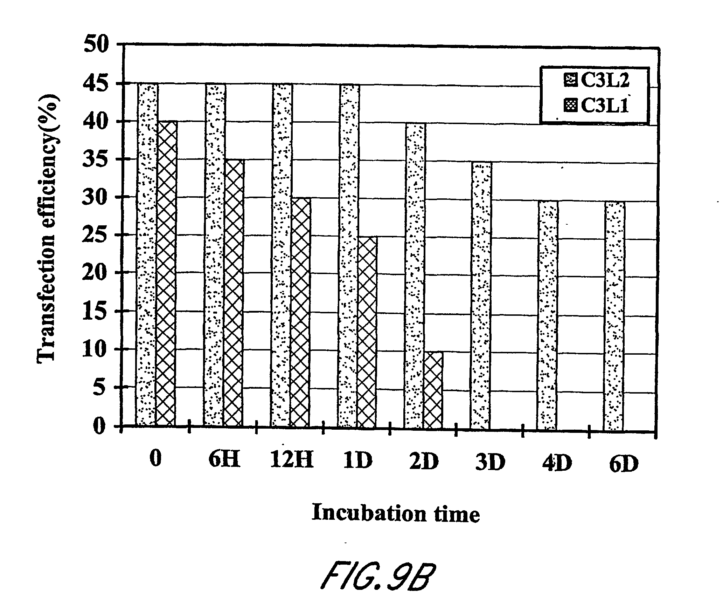

Polymers with different degradation rates can be easily obtained by simply changing different types of linkers. For example, polymers C3L1 and C3L2, were synthesized from same cationic compound, branch PEI 600D (C3), but with different hydrolyzable linkers. C3L1 is synthesized using 1,3-butanediol diacrylate (L1) and C3L2 is synthesized using 2-methyl-2,4 pentanediol diacrylate (L2). The resulting polymers showed different degradation rates in gel electrophoresis assays and transfection assays. The transfection assays showed that there is almost no change in the transfection efficiency of C3L2 polymer after 24 hours incubation with BPS at 37°C, with only a 10% decrease after 3 days incubation with PBS at 37°C, while the transfection efficiency of polymer C3L1 showed significant decreases from 40% to 25% after 24 hours and almost 0% after 3 days incubation at same conditions (Fig. 9A). The data from agarose gel electrophoresis are also consistent with the transfection assay in which there was almost no significant difference in C3L2 molecular weight (Fig. 9B, bottom), while the molecular weight of C3L1 changed at 6 hours and was almost the same compared to C3 after 4 days degradation (Fig. 9B top). The results indicate that the polymer degradation rate can be controllable and the desired degradation rate can be achieved by using a different linker.

### Example 18

### Novel Synthetic Polymer-Mediated Antisense Oligodeoxylnucleotide Delivery

The capacity of antisense ODN delivery efficiency of C4, C3, C2, C3L1, C2L3, C1L3 , BPEI25K and Lipofectamine was examined in this experiment.

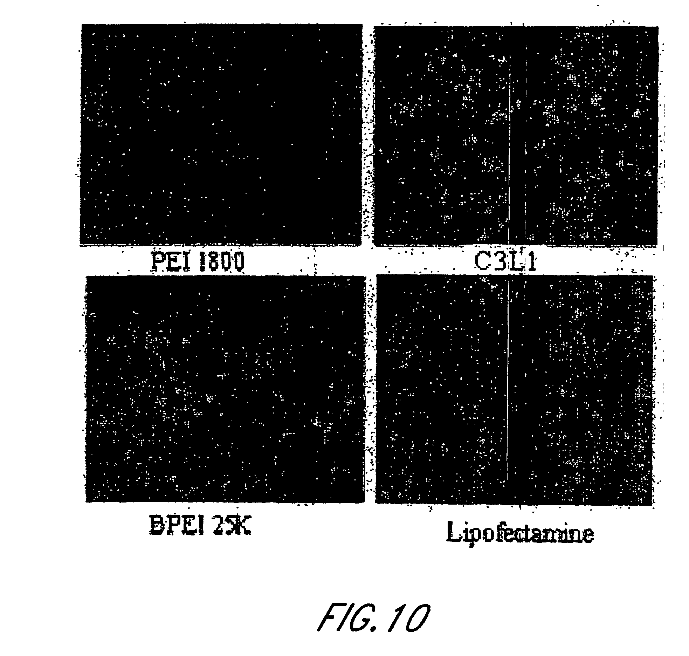

Different ratios of samples in 25µl DMEM (no serum or antibiotics) were added into 0.3µg FITC labeled ODN in 10µl DMEM (no serum or antibiotics) drop by drop and vortexed at same time. Fifteen minutes later, 150µl DMEM (no serum or antibiotics) was added to the polymer-ODN complexes and mixed. The final concentration of ODN was 250nM. HeLa 705 cells in 24-well plates were washed with PBS, and then the polymer-ODN complexes were added to the cells. FITC signal was observed under a microscope. C4, C3, C2 showed no efficiency in delivering FITC-labeled ODN. BPEI25K and Lipofectamine had high efficiency in ODN delivery. BPEI had higher delivery efficiency than Lipofectamine, and 2 hours after ODN treatment, about 60-70% of the cells showed FITC signal when the PEI/ODN ratio was 0.25µg/0.3µg. At that time, only 5-10% of cells were positive in the Lipofectamine group. Twenty-four hours after ODN delivery, more than 85% of the cells showed FITC signal, in BPEI 25k groups, while 65% of the cells showed FITC signal in the Lipofectamine group. All 3 samples showed high ODN delivery efficiency. C3L1, C2L3, C1L3 showed a little bit lower delivery efficiency than BPEI25K, and their cytotoxicities were much lower than BPEI25K. The ODN delivery efficiencies of C3L1, C2L3, C1L3 were higher than Lipofectamine. (Fig.10)

## Claims

1. A degradable cationic polymer comprising a plurality of cationic molecules and at least one degradable linker molecule connecting said cationic molecules in a branched arrangement, wherein said cationic molecules are selected from the group consisting of:
(i) a cationic compound formula 1: 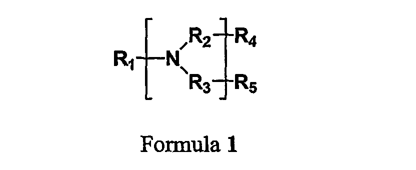 wherein:
R₁ is a hydrogen atom, an alkyl or heteroalkyl group of 2 to 10 carbon atoms, an aryl or heteroaryl group with 5 to 30 atoms, or another Formula 1;
R₂ is a hydrogen atom, an alkyl or heteroalkyl group of 2 to 10 carbon atoms, or aryl or heteroaryl group with 5 to 30 atoms;
R₃ is a hydrogen atom, an alkyl or heteroalkyl group of 2 to 10 carbon atoms, or aryl or heteroaryl group with 5 to 30 atoms;
R₄ is a hydrogen atom, an alkyl or heteroalkyl group of 2 to 10 carbon atoms, an aryl or heteroaryl group with 5 to 30 atoms, or another Formula 1;
R₅ is a hydrogen atom, an alkyl or heteroalkyl group of 2 to 10 carbon atoms, an aryl or heteroaryl group with 5 to 30 atoms, or another Formula 1;
(ii) a cationic polyamino acid; and
(iii) a cationic polycarbohydrate;
and wherein said degradable linker molecule is represented by the formula:
CLn
wherein C is a spacer moiety that is a straight or branched alkyl or heteroalkyl group of 2 to 10 carbon atoms, or aryl or heteroaryl group with 5 to 30 atoms, may contain ether, ester, amide, imide, carbonyl groups with or without heteroatoms; L is an acrylate or methacrylate moiety, and n is an integer greater than or equal to two; and wherein C and L are bound covalently.

2. The degradable cationic polymer of Claim 1, wherein said polymer is degraded by hydrolysis, enzyme cleavage, reduction, photo-cleavage, or sonication.

3. The degradable cationic polymer of Claim 1, wherein the cationic molecules are selected from the group consisting of, polyethyleneimine (PEI), polypropyleneimine (PPI), pentaethylenehexamine, N,N'-Bis(2-aminoethyl)-1,3-propanediamine, N-(2-aminoethyl)-1,3-propanediamine, N-(2-aminopropyl)-1,3-propanediamine spermine, spermidine, 1,4-Bis(3-aminopropyl) piperazine, 1-(2-aminoethyl)piperazine, tri(2-aminoethyl)amine, branched or dendrite polyamidoamine (PAMAM), poly(L-lysine) (PLL), and chitosan.

4. The degradable cationic polymer of Claim 1, wherein the linker molecule is selected from the group consisting of 1,3-butanediol diacrylate, 1,4-butanediol diacrylate, 1,6-hexanediol diacrylate, Di(ethylene glycol) diacrylate, poly(ethylene glycol) diacrylate, 2,4-pentanediol diacrylate, 2-methyl-2,4-pentanediol diacrylate, 2,5-dimethyl-2,5-hexanediol diacrylate, trimethylolpropane triacrylate, pentaerythritol tetraacrylate, Di(trimethylolpropane tetraacrylate, Dipentaerythritol pentaacrylate, and a polyester with at least three acrylate or acrylamide side groups.

5. The degradable cationic polymer of Claim 1, wherein the molecular weight of the polymer is from 500 Da to 1,000,000 Da, preferably from 2000 Da to 200,000 Da.

6. The degradable cationic polymer of Claim 1, wherein the molecular weight of the cationic compound is from 50 Da to 10,000 Da,

7. The degradable cationic polymer of Claim 1, wherein the molecular weight of the linker molecule is from 100 Da to 40,000 Da.

8. The degradable cationic polymer of Claim 1, further comprising a biomolecule complexed to the polymer.

9. The degradable cationic polymer of Claim 8, wherein the biomolecule is selected from nucleic acid, protein, peptide, lipid, and carbohydrate.

10. The degradable cationic polymer of Claim 9, wherein the nucleic acid is selected from the group consisting of DNA, single or double strand RNA, ribozyme, DNA-RNA hybridizer, and antisense DNA.

11. Use of a degradable cationic polymer of Claim 10 in the manufacture of a medicament for treating a patient in need of treatment by a method of gene therapy.

12. A method of transfecting a eukaryotic cell comprising contacting said cell with a polymer of Claim 10.

13. A method of delivering a diagnostic imaging composition to an individual comprising conjugating a diagnostic imaging composition to the degradable cationic polymer of Claim 1 and administering the conjugated polymer to the individual.

14. A polymer library comprising a plurality of degradable cationic polymers of Claim 1 wherein said polymers comprise different ratios of cationic compound to linker molecule.

15. A polymer library comprising a plurality of degradable cationic polymers of Claim 1 wherein said polymers each comprise a different linker molecule.

16. A polymer library comprising a plurality of degradable cationic polymers of Claim wherein said polymers each comprise a different cationic oligomer.

17. A biomsiarial delivery system comprising,
at least one biomolecule;
the degradable cationic polymer of Claim 1 wherein said polymer has an affinity for said biomolecule; and
at least one delivery enhancing agent, which can be internalized into a eukaryotic cell, that can facilitate receptor recognition, internalization, escape of the biomolecule from the endosome, nucleus localization, biomolecule release, or system stabilization in said eukaryotic cell.

18. The biomaterial delivery system of Claim 17 wherein said biomolecule is a nucleic acid, peptide, protein, or carbohydrate.

19. The biomaterial delivery system of Claim 18 wherein said delivery enhancing agent is coupled to said degradable cationic polymer.

20. A medical diagnostic system comprising:
an image contrast agent,
the degradable cationic polymer of Claim 1 wherein the polymer has affinity for biomolecules, and
a ligand, antibody, or agent of interest that recognizes a specific receptor of a eukaryotic cell, tissue, or organ, wherein said ligand, antibody, or agent is coupled with said degradable cationic polymer.

21. A pharmaceutical composition comprising:
a sensitizer agent that can be functionally triggered by light or other physical stimulators; and
the degradable cationic polymer of Claim 1, wherein said polymer has affinity for biomolecules.

22. The biodegradable cationic polymer of claim 10 for use in medicine.

## Patentansprüche

1. Abbaubares kationisches Polymer, das eine Vielzahl kationischer Moleküle und mindestens ein abbaubares Linkermolekül, das die kationischen Moleküle in einer verzweigten Anordnung verbindet, umfasst, wobei die kationischen Moleküle aus der Gruppe von:
(i) einer kationischen Verbindung der Formel 1: 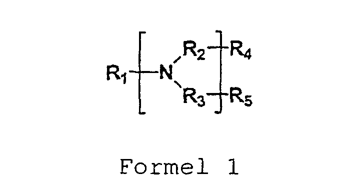 worin:
R₁ ein Wasserstoffatom, eine Alkyl-.oder Heteroalkylgruppe mit 2 bis 10 Kohlenstoffatomen, eine
Aryl- oder Heteroarylgruppe mit 5 bis 30 Atomen oder eine weitere Formel 1 bedeutet;
R₂ ein Wasserstoffatom, eine Alkyl- oder Heteroalkylgruppe mit 2 bis 10 Kohlenstoffatomen oder eine
Aryl- oder Heteroarylgruppe mit 5 bis 30 Atomen;
R₃ ein Wasserstoffatom, eine Alkyl- oder Heteroalkylgruppe mit 2 bis 10 Kohlenstoffatomen oder eine
Aryl- oder Heteroarylgruppe mit 5 bis 30 Atomen;
R₄ ein Wasserstoffatom, eine Alkyl- oder Heteroalkylgruppe mit 2 bis 10 Kohlenstoffatomen, eine
Aryl- oder Heteroarylgruppe mit 5 bis 30 Atomen oder eine weitere Formel 1 bedeutet;
R₅ ein Wasserstoffatom, eine Alkyl- oder Heteroalkylgruppe mit 2 bis 10 Kohlenstoffatomen, eine Aryl- oder Heteroarylgruppe mit 5 bis 30 Atomen oder eine weitere Formel 1 bedeutet;
(ii) einer kationischen Polyaminosäure; und
(iii) einem kationischen Polykohlenhydrat ausgewählt sind;
und wobei das abbaubare Linkermolekül durch die Formel
CLₙ
dargestellt wird, worin C eine Abstandseinheit, die eine gerade oder verzweigte Alkyl- oder Heteroalkylgruppe mit 2 bis 10 Kohlenstoffatomen oder eine Aryl- oder Heteroarylgruppe mit 5 bis 30 Atomen ist, Ether-, Ester-, Amid-, Imid-, Carbonylgruppen mit oder ohne Heteroatome enthalten kann, bedeutet; L eine Acrylat- oder Methacrylateinheit bedeutet, und n eine ganze Zahl von größer als oder gleich zwei bedeutet; und wobei C und L kovalent gebunden sind.

2. Abbaubares kationisches Polymer nach Anspruch 1, wobei das Polymer durch Hydrolyse, enzymatische Spaltung, Reduktion, Photospaltung oder Beschallung abgebaut wird.

3. Abbaubares kationisches Polymer nach Anspruch 1, wobei die kationischen Moleküle aus der Gruppe von Polyethylenimin (PEI), Polypropylenimin (PPI), Pentaethylenhexamin, N,N'-Bis(2-aminoethyl)-1,3-propandiamin, N-(2-Aminoethyl)-1,3-propandiamin, N-(2-Aminopropyl)-1,3-propandiamin, Spermin, Spermidin, 1,4-Bis(3-amino-propyl)piperazin, 1-(2-Aminoethyl)piperazin, Tri(2-aminoethylamin), verzweigtem oder dendritischem Polyamidoamin (PAMAM), Poly(L-lysin) (PLL) und Chitosan ausgewählt sind.

4. Abbaubares kationisches Polymer nach Anspruch 1, wobei das Linkermolekül aus der Gruppe von 1,3-Butandioldiacrylat, 1,4-Butandioldiacrylat, 1,6-Hexandioldiacrylat, Di(ethylenglykol)diacrylat, Poly-(ethylenglycol)diacrylat, 2,4-Pentandioldiacrylat, 2-Methyl-2,4-pentandioldiacrylat, 2,5-Dimethyl-2,5-hexandioldiacrylat, Trimethylolpropantriacrylat, Pentaerythrittetraacrylat, Di(trimethylolpropantetraacrylat, Dipentaerythritpentaacrylat und einem Polyester mit mindestens drei Acrylat- oder Acrylamidseitengruppen ausgewählt ist.

5. Abbaubares kationisches Polymer nach Anspruch 1, wobei das Molekulargewicht des Polymers 500 Da bis 1.000.000 Da, vorzugsweise 2.000 Da bis 200.000 Da beträgt.

6. Abbaubares kationisches Polymer nach Anspruch 1, wobei das Molekulargewicht der kationischen Verbindung 50 Da bis 10.000 Da beträgt.

7. Abbaubares kationisches Polymer nach Anspruch 1, wobei das Molekulargewicht des Linkermoleküls 100 Da bis 40.000 Da beträgt.

8. Abbaubares kationisches Polymer nach Anspruch 1, das ferner ein an das Polymer komplexgebundenes Biomolekül umfasst.

9. Abbaubares kationisches Polymer nach Anspruch 8, wobei das Biomolekül aus einer Nucleinsäure, einem Protein, Peptid, Lipid und Kohlenhydrat ausgewählt ist.

10. Abbaubares kationisches Polymer nach Anspruch 9, wobei die Nucleinsäure aus der Gruppe von DNA, einzel- oder doppelsträngiger RNA, Ribozym, DNA-RNA-Hybridisierungsmittel und Antisense-DNA ausgewählt ist.

11. Verwendung eines abbaubaren kationischen Polymers nach Anspruch 10 bei der Herstellung eines Medikaments zur Behandlung eines eine Behandlung benötigenden Patienten durch ein Verfahren der Gentherapie.

12. Verfahren zur Transfektion einer eukaryotischen Zelle, das das Inkontaktbringen der Zelle mit einem Polymer nach Anspruch 10 umfasst.

13. Verfahren zur Abgabe einer diagnostischen Bildgebungszusammensetzung an ein Individuum, das die Konjugation einer diagnostischen Bildgebungszusammensetzung mit dem abbaubaren kationischen Polymer nach Anspruch 1 und das Verabreichen des konjugierten Polymers an das Individuum umfasst.

14. Polymerbibliothek, die eine Vielzahl abbaubarer kationischer Polymere nach Anspruch 1 umfasst, wobei die Polymere verschiedene Verhältnisse von kationischer Verbindung zu Linkermolekül umfassen.

15. Polymerbibliothek, die eine Vielzahl abbaubarer kationischer Polymere nach Anspruch 1 umfasst, wobei die Polymere jeweils ein verschiedenes Linkermolekül umfassen.

16. Polymerbibliothek, die eine Vielzahl abbaubarer kationischer Polymere nach Anspruch 1 umfasst, wobei die Polymere jeweils ein verschiedenes kationisches Oligomer umfassen.

17. Biomaterialzufuhrsystem, das umfasst:
mindestens ein Biomolekül;
das abbaubare kationische Polymer nach Anspruch 1, wobei das Polymer Affinität für das Biomolekül hat; und
mindestens ein Zufuhrverstärkungsmittel, das in eine eukaryotische Zelle internalisiert werden kann, das Rezeptorerkennung, Internalisierung, Entkommen des Biomoleküls aus dem Endosom, Kernlokalisation, Biomolekülfreisetzung oder Systemstabilisierung in der eukaryotischen Zelle erleichtern kann.

18. Biomaterialzufuhrsystem nach Anspruch 17, wobei das Biomolekül eine Nucleinsäure, ein Peptid, Protein oder Kohlenhydrat ist.

19. Biomaterialzufuhrsystem nach Anspruch 18, wobei das Zufuhrverstärkungsmittel an das abbaubare kationische Polymer gekoppelt ist.

20. Medizinisches Diagnosesystem, das umfasst:
ein Bildkontrastmittel,
das abbaubare kationische Polymer nach Anspruch 1, wobei das Polymer Affinität für Biomoleküle hat, und
einen Liganden, Antikörper oder ein Mittel von Interesse, der bzw. das einen spezifischen Rezeptor einer eukaryotischen Zelle, eines Gewebes oder Organs erkennt, wobei der Ligand, Antikörper oder das Mittel an das abbaubare kationische Polymer gekoppelt ist.

21. Pharmazeutische Zusammensetzung, die umfasst:
ein Sensibilisierungsmittel, das durch Licht oder andere physikalische Stimulatoren funktional getriggert werden kann; und
das abbaubare kationische Polymer nach Anspruch 1, wobei das Polymer Affinität für Biomoleküle hat.

22. Das biologisch abbaubare kationische Polymer nach Anspruch 10 zur Verwendung in der Medizin.

## Revendications

1. Polymère cationique dégradable comprenant une pluralité de molécules cationiques et au moins une molécule de liaison dégradable liant lesdites molécules cationiques selon un arrangement ramifié, dans lequel lesdites molécules cationiques sont choisies dans le groupe constitué de :
(i) un composé cationique de formule 1 : 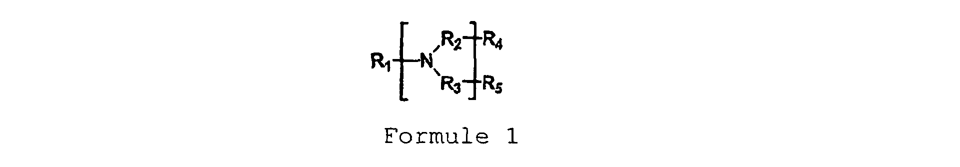 dans laquelle :
- R₁ représente un atome d'hydrogène, un groupe alkyle ou hétéroalkyle de 2 à 10 atomes de carbone, un groupe aryle ou hétéroaryle avec 5 à 30 atomes ou une autre formule 1 ;
- R₂ représente un atome d'hydrogène, un groupe alkyle ou hétéroalkyle de 2 à 10 atomes de carbone ou un groupe aryle ou hétéroaryle avec 5 à 30 atomes ;
- R₃ représente un atome d'hydrogène, un groupe alkyle ou hétéroalkyle de 2 à 10 atomes de carbone ou un groupe aryle ou hétéroaryle avec 5 à 30 atomes ;
- R₄ représente un atome d'hydrogène, un groupe alkyle ou hétéroalkyle de 2 à 10 atomes de carbone, un groupe aryle ou hétéroaryle avec 5 à 30 atomes ou une autre formule 1 ;
- R₅ représente un atome d'hydrogène, un groupe alkyle ou hétéroalkyle de 2 à 10 atomes de carbone, un groupe aryle ou hétéroaryle avec 5 à 30 atomes ou une autre formule 1 ;
(ii) un poly(acide aminé) cationique ; et
(iii) un poly(hydrate de carbone) cationique ;
et dans lequel ladite molécule de liaison dégradable est représentée par la formule :
CLn
dans laquelle C représente un groupe espaceur qui est un groupe alkyle ou hétéroalkyle linéaire ou ramifié de 2 à 10 atomes de carbone, ou un groupe aryle ou hétéroaryle avec 5 à 30 atomes, qui peut contenir des groupes éther, ester, amide, imide, carbonyle avec ou sans hétéroatomes ; L représente un groupe acrylate ou méthacrylate, et n représente un nombre entier supérieur ou égal à deux ; et dans laquelle C et L sont liés de façon covalente.

2. Polymère cationique dégradable selon la revendication 1, dans lequel ledit polymère est dégradé par hydrolyse, clivage enzymatique, réduction, photo-clivage ou sonication.

3. Polymère cationique dégradable selon la revendication 1, dans lequel les molécules cationiques sont choisies dans le groupe constitué de la polyéthylèneimine (PEI), de la polypropylèneimine (PPI), de la pentaéthylènehexamine, de la N,N'-Bis(2-aminoéthyl)-1,3-propanediamine, de la N-(2-aminoéthyl)-1,3-propanediamine, de la N-(2-aminopropyl)-1,3-propanediamine spermine, de la spermidine, de la 1,4-Bis(3-aminopropyl)pipérazine, de la 1-(2-aminoéthyl)pipérazine, de la tri(2-aminoéthyl)amine, de la polyamidoamine dendritique ou ramifiée (PAMAM), de la poly(L-lysine) (PLL) et du chitosane.

4. Polymère cationique dégradable selon la revendication 1, dans lequel la molécule de liaison est choisie dans le groupe constitué par le diacrylate de 1,3-butanediol, le diacrylate de 1,4-butanediol, le diacrylate de 1,6-hexanediol, le diacrylate de di(éthylèneglycol), le diacrylate de poly(éthylèneglycol), le diacrylate de 2,4-pentanediol, le diacrylate de 2-méthyl-2,4-pentanediol, le diacrylate de 2,5-diméthyl-2,5-hexanediol, le triacrylate de triméthylolpropane, le tétraacrylate de pentaérythritol, le tétraacrylate de di(triméthylolpropane), le pentaacrylate de dipentaérythritol et un polyester avec au moins trois groupes latéraux acrylate ou acrylamide.

5. Polymère cationique dégradable selon la revendication 1, dans lequel la masse moléculaire du polymère va de 500 Da à 1 000 000 Da, de préférence de 2 000 Da à 200 000 Da.

6. Polymère cationique dégradable selon la revendication 1, dans lequel la masse moléculaire du composé cationique va de 50 Da à 10 000 Da.

7. Polymère cationique dégradable selon la revendication 1, dans lequel la masse moléculaire de la molécule de liaison va de 100 Da à 40 000 Da.

8. Polymère cationique dégradable selon la revendication 1, comprenant en outre une biomolécule complexée au polymère.

9. Polymère cationique dégradable selon la revendication 8, dans lequel la biomolécule est choisie parmi un acide nucléique, une protéine, un peptide, un lipide et un hydrate de carbone.

10. Polymère cationique dégradable selon la revendication 9, dans lequel l'acide nucléique est choisi dans le groupe constitué par l'ADN, l'ARN double ou simple brin, le ribozyme, les hybrides ADN-ARN et l'ADN anti-sens.

11. Utilisation d'un polymère cationique dégradable selon la revendication 10 dans la fabrication d'un médicament pour traiter un patient nécessitant un traitement par un procédé de thérapie génique.

12. Procédé de transfection d'une cellule eucaryote comprenant la mise en contact de ladite cellule avec un polymère selon la revendication 10.

13. Procédé de délivrance d'une composition diagnostique pour imagerie à un individu, comprenant la conjugaison d'une composition diagnostique pour imagerie au polymère cationique dégradable selon la revendication 1 et l'administration du polymère conjugué à l'individu.

14. Banque de polymères comprenant une pluralité de polymères cationiques dégradables selon la revendication 1, dans laquelle lesdits polymères comprennent différents rapports du composé cationique à la molécule de liaison.

15. Banque de polymères comprenant une pluralité de polymères cationiques dégradables selon la revendication 1, dans laquelle lesdits polymères comprennent chacun une molécule de liaison différente.

16. Banque de polymères comprenant une pluralité de polymères cationiques dégradables selon la revendication 1, dans laquelle lesdits polymères comprennent chacun un oligomère cationique différent.

17. Système de délivrance d'un biomatériau comprenant :
- au moins une biomolécule ;
- le polymère cationique dégradable selon la revendication 1, dans lequel ledit polymère a une affinité pour ladite biomolécule ; et
- au moins un agent facilitant la délivrance, qui peut être internalisé dans une cellule eucaryote, qui peut faciliter la reconnaissance du récepteur, l'internalisation, la sortie de la biomolécule de l'endosome, la localisation dans le noyau, la libération de la biomolécule ou la stabilisation du système dans ladite cellule eucaryote.

18. Système de délivrance d'un biomatériau selon la revendication 17, dans lequel ladite biomolécule est un acide nucléique, un peptide, une protéine ou un hydrate de carbone.

19. Système de délivrance d'un biomatériau selon la revendication 18, dans lequel ledit agent facilitant la délivrance est couplé audit polymère cationique dégradable.

20. Système de diagnostic médical comprenant :
- un agent de contraste d'image,
- le polymère cationique dégradable selon la revendication 1, dans lequel le polymère a une affinité pour les biomolécules, et
- un ligand, un anticorps ou un agent d'intérêt qui reconnaît un récepteur spécifique d'une cellule eucaryote, d'un tissu ou d'un organe, dans lequel ledit ligand, anticorps ou agent est couplé audit polymère cationique dégradable.

21. Composition pharmaceutique comprenant :
- un agent sensibilisant qui peut être fonctionnellement déclenché par la lumière ou d'autres stimulateurs physiques ; et
- le polymère cationique dégradable selon la revendication 1, dans lequel ledit polymère a une affinité pour les biomolécules.

22. Polymère cationique biodégradable selon la revendication 10 pour une utilisation en médecine.
